# EUROPÄISCHE PATENTANMELDUNG

(11) **EP 4 659 739 A1**
(43) Veröffentlichungstag der Anmeldung: **10.12.2025**
(21) Anmeldenummer: 24180135.6
(22) Anmeldetag: 05.06.2024
(51) Int. Cl.: A61K 9/00, A61K 9/06, A61K 9/16, A61K 47/46

(54) **TOPISCHE FORMULIERUNG ENTHALTEND NICHT-KOVALENTE KERATIN-WIRKSTOFF-KONJUGATE ZUR THERAPEUTISCHEN ANWENDUNG**

(71) Anmelder: Rigi Therapeutics AG, 6045 Meggen (CH)
(72) Erfinder: SPORN, Christian., 06120 Halle (Salle) (DE)
(74) Vertreter: Pfenning, Meinig & Partner mbB

(57) **Zusammenfassung**

Die vorliegende Erfindung betrifft eine topische Formulierung enthaltend nicht-kovalente Keratin-Wirkstoff-Konjugate aus Partikeln des Proteins beta-Keratin und/oder deren Agglomerate aus Federn tierischen Ursprungs zur dermatologischen Anwendung. Die topische Formulierung dient der Steuerung der kutanen und/oder subkutanen und/oder systemischen Bioverfügbarkeit mindestens eines Wirkstoffes zur Therapie von Erkrankungen.

## Beschreibung

Die vorliegende Erfindung betrifft eine topische Formulierung enthaltend nicht-kovalente Keratin-Wirkstoff-Konjugate aus Partikeln des Proteins beta-Keratin und/oder deren Agglomerate aus Federn tierischen Ursprungs zur dermatologischen Anwendung. Die topische Formulierung dient der Steuerung der kutanen und/oder subkutanen und/oder systemischen Bioverfügbarkeit mindestens eines Wirkstoffes zur Therapie von Erkrankungen.

Topika stellen stofflich eine Mischung aus verschiedenen Komponenten dar, die je nach physikochemischen Eigenschaften der miteinander komplex interagierenden Bestandteile eine molekulare Ordnung bedingen. Diese Ordnung muss als ein dynamischer Gleichgewichtszustand betrachtet werden, der sich durch Änderungen in der Zusammensetzung (Zugabe oder Entzug von Bestandteilen), durch Energieaufnahme oder -abgabe (z.B. Temperatur, Licht) sowie durch chemische/-biochemische Prozesse (z.B. Löslichkeitsänderungen, pH, Metabolisierung) umstrukturiert werden kann und neue Gleichgewichtszustände ausbildet. Aus pharmazeutischer Sicht werden bestimmte Grundmuster dieser molekularen Ordnung als sogenannte Grundlagen bezeichnet und nach pharmazeutisch definierten Kriterien systematisiert.

Die inhaltlichen Bestandteile von Topika können in bestimmte Kategorien unterteilt werden, wobei diese sehr stark variieren können und z.T. nicht zwangsläufig enthalten sein müssen. Um die Zweckbestimmung des Topikums deutlich zu machen, werden der oder die aktiven Inhaltstoffe (z.B. Arzneistoffe) deklariert. Darüber hinaus sind für die molekulare Grundordnung bedeutsame Komponenten, sogenannte Matrixstoffe (z.B. Wasser, Lipide, Emulgatoren, Matrixbilder usw.) bzw. Hilfsstoffe, wie Lösungsmittel, Konsistenz- oder Farbgeber, Puffersubstanzen, Radikalfänger oder Duftstoffe zu betrachten.

Um die Effekte und Wirkungen, die ein Topikum nach der Applikation auf und im Hautorgan induziert, gezielt therapeutisch nutzen zu können, ist ein Grundverständnis für die Prozesse der Interaktion des Topikums mit dem Stratum corneum als Akzeptor Voraussetzung. Dabei ist aus dermatologischer Sicht essentiell, dass die pathologischen Veränderungen der Haut und die sich daraus ergebenden Konsequenzen für die physikochemische Interaktion analysiert und interpretiert werden müssen. Zudem sollte man sich bewusst machen, das sich die molekulare Ordnungsstruktur der Inhaltstoffe eines Topikums vor der Applikation (Applikationsmatrix) während und nach der Applikation auf bzw. in den obersten Schichten der Haut (Segregationsmatrix) einem strukturellen und damit auch funktionellen Wandlungsprozess unterliegen. Dieser wird als Metamorphose des Topikums bezeichnet, bezieht sich aber nicht nur, wie der Name suggeriert, auf einen strukturellen, sondern eben auch auf einen funktionellen Wandel, der in Abhängigkeit von den volatilen Bestandteilen der Matrix und den Umgebungsbedingungen einem dynamischen Ablauf unterliegt (dynamische Permutation), der parallel mit Diffusionsprozessen vonstattengeht. Diese komplexen Vorgänge sind alles andere als trivial, nur zum Teil vorhersehbar und essentiell für die Effekte, die durch das Topikum mit Bezug auf die Vehikelfunktion für die aktiven Inhaltsstoffe sowie die Eigenwirkung von Inhaltstoffen auf die Funktionsstruktur der oberen Hautschichten vermittelt werden.

Die pharmakokinetischen Vorgänge werden durch drei miteinander funktionell überlappende Abläufe bestimmt. Unmittelbar nach der Applikation eines Topikums auf die Hautoberfläche werden der darin gelöste oder suspendierte Wirkstoff bzw. weitere aktive Inhaltstoffe aus der Vehikelmatrix freigesetzt (liberiert). Für eine potentielle Wirkung steht nur der liberierte und bioverfügbare Wirkstoffanteil zur Verfügung. Die Liberationsrate wird dabei wesentlich durch die Eigenschaften der Vehikelmatrix selber sowie der physikochemischen Eigenschaften des Applikationsareals (Akzeptor) bestimmt. Da der Zustand des Applikationsareals sich entsprechend der Akuität der Indikation bzw. inter- und intraindividuell stark variieren kann, werden Schwankungen der kutanen Bioverfügbarkeit eines Wirkstoffs deutlich, die Einfluss auf den gewünschten therapeutischen Effekt begründen. Eine besondere Bedeutung kommt in diesem Zusammenhang der Beschaffenheit und molekularen Ordnung des Stratum corneums zu, da dieses als Kontaktschicht zur Vehikelmatrix den unmittelbaren Akzeptor für eine Wirkstoffaufnahme bildet. Dieser Umstand wird im pharmakokinetischen Kontext als "Reservoirfunktion des Stratum corneums" bezeichnet. Der Wirkstofftransfer aus der Vehikelmatrix in den Akzeptor wird darüber hinaus zeitlich stark durch die effektive Kontaktzeit des Topikums mit dem Stratum corneum limitiert. Meist stehen dafür nur wenige Minuten zur Verfügung, da Rückstände der Vehikelmatrix durch Kleidung oder anderweitig rasch der Hautoberfläche entzogen werden. Für die Bioverfügbarkeit im Zielkompartiment ist nach der Liberation eine Distribution des Wirkstoffs in die Mikrokompartimente der jeweiligen Hautschichten und physikochemischen Zonen innerhalb einzelner Kompartimente notwendig, die als Penetration bezeichnet wird. Dieser Verteilungsprozess wird wesentlich durch passive Diffusion realisiert, die den Gesetzmäßigkeiten der Fick'schen Diffusionsgesetze folgen. Dabei spielen Konzentrationsunterschiede, Diffusionsfläche, Diffusionsstrecke und die Diffusionseigenschaften der einzelnen Kompartimente (Diffusionskoeffizient) die entscheidende Rolle. In Abhängigkeit von der Zusammensetzung der Vehikelmatrix ist aber auch die thermodynamische Aktivität bzw. Vorgänge der Konvektion in Abhängigkeit des hydrodynamischen Druckgradienten (Hagen-Poiseuille'sches Gesetz) von Bedeutung. Als Permeation wird der Prozess bezeichnet, der die jeweilige oder alle Hautschichten passiert hat und in angrenzende Gewebeschichten diffundiert ist. Der in die gefäßführenden Schichten penetrierte Wirkstoffanteil wird anteilig durch Absorption insbesondere über das mikrohämovaskuläre System sowie metabolisch durch sessile Zelltypen eliminiert.

Als wesentlicher Akzeptor für topisch applizierte Arzneistoffe fungiert das Stratum corneum (SC) der Epidermis, welches aus mehreren Schichten von keratinisierten Korneozyten, die vom *cornified envelope* ummantelt und durch korneodesmosomale Strukturen verbunden in eine komplexe Lipidmatrix aus flüssigkristallinen, lamellaren Strukturen eingebettet sind, besteht. Die Lipidfraktion enthält im Wesentlichen Ceramide (CERs), langkettige freien Fettsäuren (FFS), Cholesterol (CHOL) und Triglyceriden (TG). Diese bilden in der Interaktion mit Wasser ein komplexes Membrannetzwerk, welches als morphologisches Äquivalent zur Barrierefunktion verstanden wird. CERs bilden dabei, wegen ihrer anisotropen Molekülstruktur und den dadurch begründeten besonderen physikochemischen Eigenschaften, das Rückgrat des Membrannetzwerks, während die anderen lipophilen Bestandteile die Membraneigenschaften, wie Fluidität, Permeabilität und Stabilität modulieren.

Der Diffusionsprozess in und durch das Stratum corneum erfolgt über verschiedene physikochemische Passagewege. Die bedeutendste Route ist dabei der interzelluläre Passageweg, der wiederum eine hydrophile und lipophile Diffusionsstrecke bietet. Der hydrophile Passageweg wird durch die hydrophilen Kopfgruppen der Ceramide und die damit interagierenden Wassermoleküle in einer membranartigen Molekülordnung realisiert. Beim lipophilen Passageweg dienen die beweglichen lipophilen Seitenketten der Ceramide als penetrationsbahnendes Milieu.

Sowohl die pathogenetischen Gegebenheiten einer zu behandelnden Dermatose, als auch die pharmakodynamischen Eigenschaften einer Wirksubstanz bestimmen das galenische Zielkompartiment, welches für die Optimierung des Konzentrations-Zeit-Profils der Wirksubstanz, also der Effektivität und Dynamik des Diffusionsprozesses maßgeblich sind. Um eine optimierte therapeutische Effizienz eines Topikums zu erreichen, muss deshalb die Gesamtheit des Diffusionsmilieus am Applikationsareal und dessen physikochemischen Bedingungen in die Betrachtung einbezogen werden. Im Zentrum der Betrachtungen steht aber das galenische Konzept eines Topikums, welches letztlich die Grundvoraussetzung für das gezielte Erlangen einer kutanen Bioverfügbarkeit des oder der applizierten Wirkstoffe in den Schichten der Haut bewirkt, in der die Wirkung induziert werden soll (Zielkompartiment).

Je nach therapeutischem Anliegen, physikochemischen Eigenschaften des Arzneistoffs und galenischen Ausgangsbedingungen kann sich die Notwendigkeit sowohl einer Penetrationspromotion als auch einer Penetrationsretardierung durch gezielte Einflussnahme auf den Freisetzungs-, Permeations- bzw. Penetrationsprozess des Arzneistoffs aus der topischen Formulierung ergeben. Dazu können unterschiedliche Strategien angewendet werden, die sich grob in chemische (penetrationsförderne Chemikalien), biochemische (Prodrugs, chemische Molekülmodifikation, Enzyminhibition, kolloidales System usw.) und physikalische Verfahren (Hydratisierung, Phono- oder lontophorese, Wärmeeintrag, Laserenergieeintrag, Porationsverfahren usw.) unterteilen lassen. Praktisch finden vor allem chemische Modifikationen Verwendung, die je nach den o.g. Ausgangsbedingungen ausgewählt und optimiert werden. Dabei werden mit Bezug auf die Penetration Verstärker (*enhancer*)*,* Beschleuniger (*accelerants*)*,* Wirkverstärker (*adjuvants*)*,* Anreicherungsinduktoren (*sorption promoters*) bzw. Penetrationshemmer (*inhibitors*) oder Penetrationsverzögerer (*retarder*) unterschieden. Die damit verbundenen Substanzen und deren chemische Reaktionsgruppen (z.B. Sulfoxide, Alkohole, Fettsäuren, Fettsäureester, Polyole, Amide, Tenside, Terpene, Alkanone oder organische Säuren) sind vielfältig.

Der Einsatz von Proteinen als Arzneistoffträger ist bisher nur für die systemische Applikation bekannt und etabliert. Hierbei werden vorzugsweise therapeutische Antikörper in Form von kovalenten Antikörper-Wirkstoff-Konjugaten (antibody-drug-conjugates, ADCs) eingesetzt. Für die topische Applikation ist der Einsatz derartiger Konstrukte wegen des hohen Molekulargewichts der Antikörper bzw. anderer Proteine und wegen der hohen Bindungsaffinität durch kovalente Bindungen, vermittelt durch Verbindungsmoleküle (*linker*)*,* nicht möglich. Gleiches gilt für die neuerdings verwendeten Peptid-Wirkstoff-Konjugate (Peptide-drug conjugates, PDCs).

Der Einsatz von monomerem beta-Keratin für die gezielte Beeinflussung der Pharmakokinetik von topisch applizierten Arzneistoffen zur Therapie einer definierten medizinischen Indikation ist bisher nicht bekannt.

Ausgehend hiervon war es Aufgabe der vorliegenden Erfindung zu zeigen, dass Modellsubstanzen über nicht-kovalente Bindungen eine bimolekulare Wechselwirkung mit entsprechenden Molekülabschnitten des monomeren Keratins eingehen und ohne Verwendung spezifischer Verbindungsmoleküle (*linker*) ein nicht-kovalentes Protein-Wirkstoff-Konjugat bilden.

Diese Aufgabe wird mit der topischen Formulierung mit den Merkmalen des Anspruchs 1 sowie die Verwendung gemäß Anspruch 13 gelöst. Die weiteren abhängigen Ansprüche nennen bevorzugte Ausführungsformen.

Erfindungsgemäß wird eine topische Formulierung, die mindestens ein nicht-kovalentes Protein-Wirkstoff-Konjugat des monomeren Proteins beta-Keratin und/oder deren Agglomerate aus Federn tierischen Ursprungs und mindestens einen Wirkstoff enthält, zur therapeutischen Anwendung bereitgestellt.

Unter nicht-kovalenten Konjugaten sind Konjugate zu verstehen, bei denen nicht über einen Linker eine kovalente Bindung zwischen Wirkstoff und Protein vorliegt, sondern die Bindung auf intermolekularen Anziehungskräften wie wie Van-der-Waals-Wechselwirkungen oder Wasserstoffbrückenbindungen beruht.

Die vorliegende Erfindung betrifft somit den Einsatz von Keratinpartikeln des Proteins beta-Keratin, die durch chemische Denaturierung aus Vogelfedern als Protein beta-Keratin extrahiert und in flüssigen oder halbfesten Zubereitungen zur epikutanen Applikation eingesetzt werden.

In kosmetischen Präparaten eingesetzte Keratin-Peptide aus hydrolysiertem Keratin von Tierhaaren (z.B. Schafswolle) ist in der Sequenz kurzkettig und weist andere Eigenschaften auf (z.B. Molekülgröße, Quellverhalten) als das native, hoch-sequente Keratin-Protein, welches hier erstmalig gewonnen werden konnte. Im Unterschied zu bisherigen Verfahren wurde auf eine Hydrolyse zur Extraktion verzichtet und stattdessen das Protein über das Brechen der Disulfidbrücken für die Extraktion zugänglich gemacht. Dadurch bleiben die Primärstruktur und die Sekundärstruktur des Proteins Keratin als beta-Faltblatt erhalten.

Diese bandförmigen Keratinmonomere inserieren in die im Stratum corneum vorhandenen Ceramidmembranen und bedingen dadurch deren dynamische Stabilisierung. Durch diese Einbindung wird ein residenter Zustand dieser Proteine im Stratum corneum bedingt, der sowohl durch die Interaktion mit den hydrophilen Kopfgruppen der Ceramide, als auch mit den lipophilen Seitenketten der Ceramide vermittelt wird. Proteinseitig wird dies durch den amphiphilen Charakter von monomerem Keratin und der besonderen Sequenz von hydrophilen und lipophilen Aminosäuren im Molekülgerüst ermöglicht.

Die Federn tierischen Ursprungs sind vorzugsweise ausgewählt aus der Gruppe bestehend aus Vogelfedern, insbesondere Federn von Hühnern, Gänsen, Puten, Enten, Truthähnen, Fasanen, Straußen, Nandus, Emus, Wachteln und Mischungen hiervon.

Als Keratinquelle wurden verschiedene tierische Materialien ausgewählt, die aber aufgrund ihres Proteinvernetzungsgrades unterschiedlich geeignet erscheinen. Nach Voruntersuchungen mit unterschiedlichen Keratin-enthaltenden Biomaterialien zeigte sich eine besondere Eignung von Vogelfedern. Neben praktischen Aspekten der Verfügbarkeit und einfacher Verarbeitung wurden vor allem biochemische Aspekte erarbeitet, die eine präferenzielle Verwendung von Federn begründen. Das in Federn enthaltene Keratin entspricht vorwiegend beta-Keratin, welches als Polypeptidkette mit einer beta-Faltblattstruktur ausgestattet ist, aus Filamenten von 3-4 nm besteht und eine molekulare Masse von ca. 10-22 kDa aufweist. Im Unterschied zu alpha-Keratin bei Säugetieren finden sich bei beta-Keratin wenige, aber funktionell bedeutende Unterschiede in der Primärsequenz. So bildet beta-Keratin weniger Makrofibrillen als alpha-Keratin und zeigt ein regelmäßigeres Ordnungs- und Packungsverhalten. Diese Unterschiede bieten somit günstigere Voraussetzungen für eine Standardisierung der Keratinisolierung und der daraus resultierenden Produkteigenschaften. Dabei gilt es insbesondere die häufig praktizierte Hydrolysierung von Keratin zu vermeiden, um intakte, monomere Keratinproteine zu isolieren und die Bildung von Keratinbruchstücken, Aminosäuren und Peptide gezielt zu vermeiden.

Es ist bevorzugt, dass die Keratinpartikel ein mittleres Molekulargewischt im Bereich von 5 bis 50 kDa, bevorzugt von 10 bis 30 kDa, gemessen mittels Natriumdodecylsulfat-Polyacrylamidgelelektrophorese (SDS-PAGE), aufweist.

Es ist bevorzugt, dass das Protein beta-Keratin in seiner Sekundärstruktur als beta-Faltblatt vorliegt. Im Unterschied zum alpha- (= soft fiber) Keratin zeigt beta- (= hard fiber) Keratin einen hohen Anteil an eng zusammengedrehter und durch Disulfidbrücken stabilisierter beta-Faltblattstrukturen, die eine hohe Stabilität des hard fiber-Keratins gewährleisten.

Es ist bevorzugt, dass die Keratinpartikel und/oder deren Agglomerate in der topischen Formulierung eine Partikelgröße im Bereich von 10 bis 120 µm, bevorzugt von 25 bis 100 µm, gemessen mittels dynamischer Lichtstreuung (nach DIN ISO 22412:2018-09) (Gerät Zetasizer ZEN3600, Malvern Panalytical Instruments), aufweisen.

Eine weitere bevorzugte Ausführungsform sieht vor, dass die topische Formulierung Additiva enthält. Diese sind bevorzugt ausgewählt aus der Gruppe bestehend aus
- Aminosäuren, Peptide, Proteinen,
- Kohlenhydraten, insbesondere Saccharose, Lactose, Glucose, Fructose, Mannitol, Sorbitol und Süßstoffen wie Saccharin-Natrium, Natriumcyclamat, Aspartam, Stärke und modifizierte Stärke, Cyclodextrine und/oder Mischungen hiervon,
- Ionische Tenside, wie anionische bzw. polyanionische Tenside, insbesondere Natriumdodecylsulfat, Natriumcetylstearylsulfat, Cetylstearylalkohol (emulgierend), Natriumdioctylsulfosuccinat und/oder kationische Tenside, insbesondere Metallseifen und/oder Mischungen hiervon,
- Nichtionische Tenside, insbesondere Fettalkohole und Sterole, Sortitanfettsäureester, Polyoxyethylen-Sorbitanfettsäureester, Polyoxyethylen-Fettsäureglyceride, Macrogol-1000-glycerolmo-nofettsäureester, Polyoxyethylen-Fettsäureester, Polyoxyethylen-Fettalkoholether, Glycerolfettsäureester, Saccharosefettsäureester, Poloxamere und/oder Mischungen hiervon,
- Gelbildner, insbesondere Polyacrylate, Poloxamer, Cellulosederivate, wie Methycellulose, Methylhydroxyproylcellulose Hydroxypropylcellulose, Hydroxyethylcelluloseund/oder Ethylcellulose, Carmellose-Natrium und/oder Mischungen hiervon,
- Verdickungsmittel, insbesondere Tragant, Xantham, arabisches Gummi, Guargalactomannan, Alginate, Bentonit, und/oder Mischungen hiervon,
- Filmbildnern, insbesondere Methacrylsäure-Acrylaten, Polyvidon, Polyvinylalkohol und/oder Mischungen hiervon,
- Polymeren insbesondere Macrogole, Gelatine und/oder Mischungen hiervon,
- Konservierungsstoffe, insbesondere Methyl-4-hydroxybenzoat, Propyl-4-hydroxybenzoat, Sorbinsäure, Kaliumsorbat, Propylenglykol und/oder Mischungen hiervon,
- Antioxidativa, insbesondere Tocopherolacetat, Carotinoide, Flavonoide, Ascorbinsäure und/oder Mischungen hiervon,
- Duftstoffe
- Puffersubstanzen, insbesondere Natriumlactat, Glycolsäure und/oder Mischungen hiervon.

Es ist weiter bevorzugt, dass die topische Formulierung pharmazeutische Wirk- und Hilfsstoffe enthält, wobei die pharmazeutisch wirksame Verbindung ausgewählt ist aus einem oder mehreren Wirkstoffen vorzugsweise, aber nicht ausschließlich, aus der Gruppe der Immunsuppressiva oder Immunmodulatoren, Antiphlogistika, Antiallergika, Antihistaminika, Antibiotika, Antimykotika, Virustatika, Antiproliferativa, Antineoplastika, Keratolytika, Haarwuchsmitteln, Nageltherapeutika, Antipruriginosa, Lokalanästhetika, Analgetika, Rheologika und Statinen.

Es ist weiter bevorzugt, dass die topische Formulierung pharmazeutische Wirk- und Hilfsstoffe enthält, wobei die pharmazeutisch wirksame Verbindung ausgewählt ist aus einem oder mehreren Wirkstoffen zur Behandlung vorzugsweise, aber nicht ausschließlich der Psoriasis, Ekzeme, Allergien, Akne, Rosazea und andere entzündliche Hautzustände, benigne, semimaligne oder maligne Hauttumoren, Infektionen durch Pilze, Bakterien oder Viren, Infektion oder Infestation durch Protozoen oder Parasiten, Differenzierungsstörungen der Epidermis, Erkrankungen der Schleimhäute oder Übergangsschleimhaut, Kopfhauterkrankungen, Erkrankungen der Hautanhangsgebilde, Störungen der hämo- oder lymphovaskulären Perfusion, Störungen des Nervensystems, insbesondere Juckreiz- und Schmerzzustände, Bindegewebserkrankungen und Verhornungsstörungen. Hierzu zählen insbesondere alle Verbindungen, die in der Liste der Stoffe und Zubereitungen nach § 1 Nr. 1 AMVV aufgeführt sind (Stand 21.02.2020, zugänglich unter: https://www.bfarm.de/Shared-Docs/Downloads/DE/Arzneimittel/Pharmakovigilanz/Gremien/Verschreibungspflicht/liste_stoffe_zubereitungen.pdf?_blob=publicationFile&v=31).

Es ist bevorzugt, dass das mindestens eine in der Formulierung enthaltene nicht-kovalente Keratin-Wirkstoffkonjugat in präformierte Membranen von Kolloiden, insbesondere Liposomen und/oder Cerosomen und/oder Aphrone integrierbar ist.

Eine weitere bevorzugte Variante sieht vor, dass das mindestens eine in der Formulierung enthaltene nicht-kovalente Keratin-Wirkstoffkonjugat in die Hüllmembran von extrahierten extrazellulären Vesikeln, insbesondere Exosomen integrierbar ist.

Eine weitere bevorzugte Variante sieht vor, dass das in der Formulierung enthaltene mindestens eine nicht-kovalent gebundene Keratin-Wirkstoff-Konjugat in Nanopartikel aus vollständig oder anteilig amphiphilen Lipiden, insbesondere Phospholipiden oder/und Ceramiden bestehende Nanopartikeln integrierbar ist.

Es ist bevorzugt, dass die dermatologische Anwendung an Lebewesen, insbesondere an Menschen und/oder Tieren erfolgt, insbesondere für die Haut, Schleimhaut oder Hautanhangsgebilde, bevorzugt Nägeln und Haaren, wobei die dermatologische Anwendung bevorzugt die Behandlung von Erkrankungszuständen der Haut, Schleimhaut und Hautanhangsgebilde betrifft und/oder zur transdermalen Applikation von Wirkstoffen zur Therapie von Systemerkrankungen dient.

Die erfindungsgemäßen topischen Formulierungen liegen bevorzugt vor als
- gasförmige Grundlagen, insbesondere Aerosole und/oder Gasaphronen
- flüssige Grundlagen, insbesondere Lösungen, Emulsionen, Suspensionen, Schäume und/oder Kolloiden,
- halbfeste Grundlagen, insbesondere Salben, Cremes, Gele, Pasten, Kolloiden und/oder Suppositorien oder
- feste Grundlagen, insbesondere Pulver, Puder, Tabletten, Granulate, Pellets, Kapseln, Pflaster, Wundauflagen und Verbandsmittel, Textilfasern und/oder Inserts.

Die vorliegende Erfindung betrifft somit den Einsatz von Keratinpartikeln des Proteins beta-Keratin, die durch chemische Denaturierung aus Vogelfedern als Protein Keratin extrahiert und in flüssigen oder halbfesten Zubereitungen zur epikutanen Applikation eingesetzt werden.

Erfindungsgemäß werden die Keratinpartikel aus Federn tierischen Ursprungs hergestellt nach einem Verfahren, bei dem
a) eine Extraktion des Proteins beta-Keratin, insbesondere beta-Keratin in seiner Sekundärstruktur als β-Faltblatt, in einer eine chemische Denaturierung auslösenden Extraktionslösung enthaltend mindestens ein Denaturierungsagens, mindestens eine Base, mindestens ein Reduktionsmittel und mindestens eine Puffersubstanz erfolgt,
b) die Extraktionslösung aus Schritt a) einer Filtration unterzogen wird, bei der eine kolloidale Lösung der Keratinpartikel des Proteins beta-Keratin erhalten wird,
c) die kolloidale Lösung aus Schritt b) per Gefriertrocknung, Sprühtrocknung, Vakuumtrocknung, Lufttrocknung, Wärmetrocknung, Infrarottrocknung und/oder Mikrowellentrocknung getrocknet wird unter Erhalt eines Keratinpartikel enthaltenden Pulvers.

Bei der Gewinnung von Keratin aus Wollhaaren werden alpha-und beta-Keratin gewonnen, sogenanntes "soft fiber" (alpha) oder "hard fiber"(beta)-Keratin, also zwei unterschiedliche Arten des Keratins. Die hier durchgeführte Extraktion mit Harnstoff (Urea), führt im alkalischen Bereich dazu, dass die native Form des beta-Keratins als gesamtes Protein aus den Federn gewonnen wird und so den hohen Anteil an seiner Sekundärstruktur, dem β-Faltblatt, behält, der die hohe Wasserbindekapazität dieses Keratins bedingt. Beta-Keratin wird insbesondere aus Vogelfedern gewonnen. Es ist reich an den Aminosäuren Glycin und Alanin und besitzt wenig Cystein, Prolin und Hydroxyprolin.

Dabei wird die Interaktion des Keratins mit hydrophilen Substanzen als Reservoir zur gezielten Beeinflussung deren Pharmakokinetik genutzt. Durch die Kombination des Keratins mit Wasser, Aminosäuren, hygroskopischen Substanzen, Peptiden und/oder Proteinen soll zudem eine Substitution der physikalischen Barriere erzielt werden.

Vorzugsweise weist die Extraktionslösung einen pH-Wert von 8 bis 13, bevorzugt von 9 bis 12 und besonders bevorzugt von 10 bis 11 auf.

Es ist bevorzugt, dass die Keratinpartikel und/oder deren Agglomerate in der kolloidalen Lösung eine Partikelgröße im Bereich von 10 bis 120 nm, bevorzugt im Bereich von 25 bis 100 nm, gemessen mittels dynamischer Lichtstreuung (nach DIN ISO 22412:2018-09) (Gerät Zetasizer ZEN3600, Malvern Panalytical Instruments), aufweisen.

Die Keratinpartikel in Pulverform weisen eine Partikelgröße im Bereich von 1 bis 50 µm, bevorzugt von 10 bis 30 µm, gemessen mittels dynamischer Lichtstreuung (nach DIN ISO 22412:2018-09) (Gerät Zetasizer ZEN3600, Malvern Panalytical Instruments), auf. Die Agglomerate der Keratinpartikel in Pulverform weisen eine Agglomeratgröße im Bereich von 10 bis 500 µm, bevorzugt von 30 bis 250 µm, gemessen mittels dynamischer Lichtstreuung (nach DIN ISO 22412:2018-09) (Gerät Zetasizer ZEN3600, Malvern Panalytical Instruments), auf.

Es ist bevorzugt, dass das mindestens eine Denaturierungsagens ausgewählt ist aus der Gruppe bestehend aus Harnstoff, Thioharnstoff, Guanidinhydrochlorid, Natriumdodecylsulfat (SDS) und Mischungen hiervon.

Es ist weiter bevorzugt, dass die mindestens eine Base ausgewählt ist aus der Gruppe bestehend aus Natriumhydroxid, Kaliumhydroxid und Mischungen hiervon.

Das mindestens ein Reduktionsmittel ist vorzugsweise ausgewählt aus der Gruppe bestehend aus β-Mercaptoethanol, Cysteaminen, Cysteine, Glutathion, Natriumdisulfit, Natriumsulfid, Natriumhydrogensulfit, Natriumdithionit, Natriumthiosulfat, Dithiotreitol (DTT), Thioglykolsäure und ihre Salze, Thioharnstoff, Tricarboxyethylphosphan (TCEP) und andere Phosphane, Ammoniumchlorid und Mischungen hiervon.

Es ist bevorzugt, dass die mindestens eine Puffersubstanz ausgewählt ist aus der Gruppe bestehend aus Tris(hydroxymethyl)aminomethan, Natriumdodecylsulfat (SDS), Tris/Salzsäure (HCl), Ethylendiamintetraessigsäure (EDTA)/Tris, Kaliumchlorid-Natriumhydroxid (KCI-NaOH), Natriumhydrogencarbonat (NaHCO₃), Dithiotreitol (DTT)/Tris und Mischungen hiervon.

Es ist bevorzugt, dass die Extraktionslösung mindestens eine der folgenden chemischen Komponenten enthält:
- mindestens ein Oxidationsmittel ausgewählt aus der Gruppe bestehend aus Wasserstoffperoxid, Kaliumpermanganat, Natriumperborat, Peroxyessigsäure, Perameisensäure und Mischungen hiervon,
- mindestens eine Säure ausgewählt aus der Gruppe bestehend aus Salpetersäure, salpetrige Säure, hypo-und hyperhalogenige Säuren und Mischungen hiervon,
- mindestens ein ionisches Agens ausgewählt aus der Gruppe bestehend aus 1-Butyl-3-methyl-imidazolium (BMIM)-chlorid, 1-Butyl-3-methyl-imidazolium (BMIM)-Bromid, 1-Butyl-3-methyl-imida-zolium (BMIM)-tetrafluoroborat, Amidchlorid und Mischungen hiervon.

Eine bevorzugte Ausführungsform des erfindungsgemäßen Verfahrens sieht vor, dass bei der Extraktion in Schritt a) mindestens einer der folgenden Schritte erfolgt:
- eine mechanische Zerkleinerung, insbesondere durch Zermahlen via Ultraschall, bevorzugt im Frequenzbereich von 20 bis 50 Hertz, mit einer Kugelmühle und/oder mit einer Schneidmühle, bevorzugt Ultra-Turrax, wobei die mechanische Zerkleinerung nach Siebanalyse bis auf eine Partikelgröße (d50) von 0,1 bis 5,0 mm, bevorzugt 0,2 bis 1,0 mm, erfolgt,
- eine thermische Denaturierung, insbesondere bei Temperaturen von 70°C bis 150°C, und/oder elektrochemische Denaturierung,
- eine Fällung der Extraktionslösung aus Schritt a), insbesondere ausgelöst durch eine pH-Änderung, Zugabe eines Co-Solvens und/oder eines Salzes,
- eine mikrobielle und enzymatische Extraktion über
   - gram negative Bakterien ausgewählt aus der Gruppe bestehend aus *Stenotrophomonas* sp., *Chrysebacterium* sp., *Vibrio* sp. und Mischungen hiervon,
   - gram positive Bakterien ausgewählt aus der Gruppe bestehend aus *Bacillus sp., Kocuria rosea* und Mischungen hiervon
   - saprophytische und/oder parasitische Pilze und/oder
   - Mischungen hiervon,
- Behandlung mit Mikrowellenstrahlung, insbesondere Mikrowellenstrahlung bis zu 960 Watt und 2450 Hertz,
- Einsatz von Stromexplosion und/oder überkritischem Wasser und/oder.
- Kombinationen hiervon.

Es ist bevorzugt, dass das Protein beta-Keratin in seiner Sekundärstruktur als β-Faltblatt vorliegt. Im Unterschied zum alpha- (= soft fiber) Keratin zeigt beta- (= hard fiber) Keratin einen hohen Anteil an eng zusammengedrehter und durch Disulfidbrücken stabilisierter β-Faltblattstrukturen, die eine hohe Stabilität des hard fiber-Keratins gewährleisten.

Vorzugsweise erfolgt die Filtration über Dialyse und/oder Ultrafiltration (Cross-Flow-Filtration).

Es ist bevorzugt, dass zur Stabilisierung der kolloidalen Lösung mit/ohne Wirk-und/oder Hilfsstoffe diese als liposomale Systeme in uni- oder multilamellare Vesikel unterschiedlicher oder einheitlicher Größe eingebaut sind durch Zusatz von amphiphilen Molekülen. Diese amphiphilen Moleküle sind bevorzugt ausgewählt aus der Gruppe bestehend aus:
- Phospholipiden, bevorzugt Lecithin, DODAB, DPPC, DSPC, DSTAP und/oder Mischungen hiervon,
- kationischen Lipiden, bevorzugt ALC-0315,
- PEGylierte Lipide, bevorzugt ALC-0159
- Prostaglandine und -modifikationen, bevorzugt PGE1, PGD2, PGE2, 15-keto PGE1 und/oder Mischungen hiervon,
- Ceramiden, bevorzugt ausgewählt aus der Gruppe bestehend aus Ceramiden der Kopfgruppen NS, NH, NP, NDS, AS, AH, ADS, AP der Kettenlängen C₁₀ bis C₂₆ und/oder Mischungen hiervon,
- Ceramiden, bevorzugt ausgewählt aus der Gruppe bestehend aus Ceramiden der Kopfgruppen EOS, EOH, EOP der Kettenlängen C₁₀ bis C₃₂ und/oder Mischungen hiervon,
- Cholesterol, Cholesterolderivaten und/oder Mischungen hiervon,
- Fettsäuren, bevorzugt ausgewählt aus der Gruppe bestehend aus Fettsäuren der Kettenlängen C₁₀ bis C₃₂ und/oder Mischungen hiervon.

Anhand der nachfolgenden Figuren und des Beispiels soll der erfindungsgemäße Gegenstand näher erläutert werden, ohne diesen auf die hier gezeigten spezifischen Ausführungsformen einschränken zu wollen.
- Fig. 1: zeigt anhand eines Diagramms die prozentuale reepithelisierte Fläche zweier Zelltypen (HaCaT (gelb) und NHDF-Zellen (blau) nach 48-stündiger Behandlung
- Fig. 2: zeigt Querschnitte der oberen Hautschichten nach Behandlung mit einer Formulierung mit 5 % kolloidaler Keratin-Lösung in DAC-Basis-creme
- Fig. 3: zeigt Raman-Spektren im Bereich der Wellenzahl von 750 cm⁻¹ bis 3000 cm⁻¹ für die Lipide (rote Linie), das Zytoplasma (grüne Linie), die Nuklei (blaue Linie) und die kolloidale Keratin-Lösung (graue Linie)
- Fig. 4: zeigt Raman-Spektren für die Lipide, die Nuklei und das Zytoplasma der HaCaT-Zellen in allen drei Dimensionen aufgenommen und im Overlay
- Fig. 5: zeigt ein Konzentrations-Zeit-Profil von Mometasonfuroat (in µmol/l) in ex vivo Humanhaut ohne und mit Keratin-Konjugation
- Fig. 6: zeigt ein Konzentrations-Zeit-Profil von Mometasonfuroat (in µmol/l) in ex vivo Humanhaut ohne Keratin-Konjugation im Vergleich zu Vor- und Nachbehandlung mit einer Keratin-haltigen Formulierung

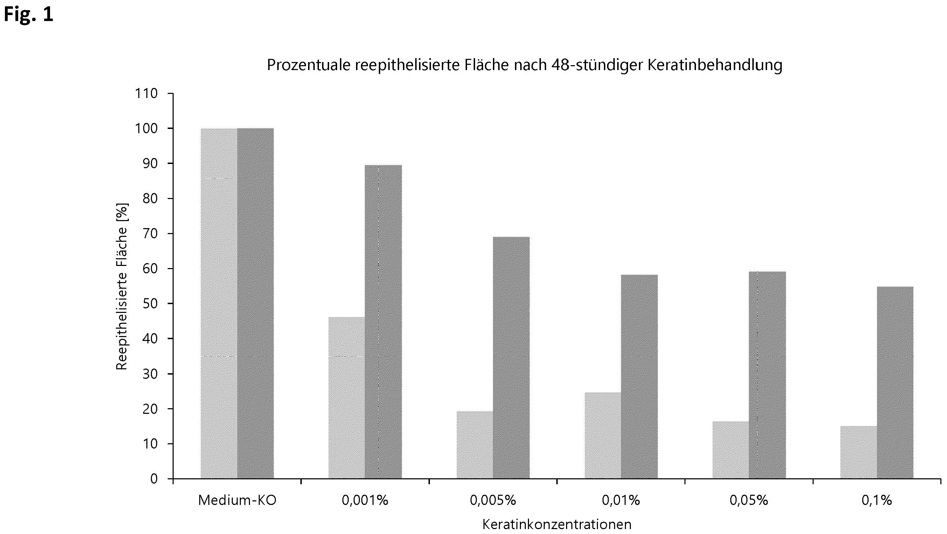

In Fig.1 ist die prozentuale reepithelisierte Fläche der HaCaT- (gelb) und NHDF-Zellen (blau) nach 48-stündiger Behandlung dargestellt. Dabei wurden Messungen mit unterschiedlichen Mengen an Keratinpartikeln durchgeführt, d.h. mit 0,001 %, 0,005 %, 0,01 %, 0,05 % und 0,1 % Keratinpartikel. Die Zellmigration (Wundheilungseffekt) wurde nach der Zugabe der kolloidalen Keratin-Lösung getestet. Als Kontrolle wurden die Zellen ohne Keratin-Zusatz in ihrem entsprechenden Medium kultiviert (Medium-KO). Die Daten zeigen, dass die untersuchten Keratinpartikel durch mechanische Hemmung konzentrationsabhängig die Migration der untersuchten Zelltypen behindern.

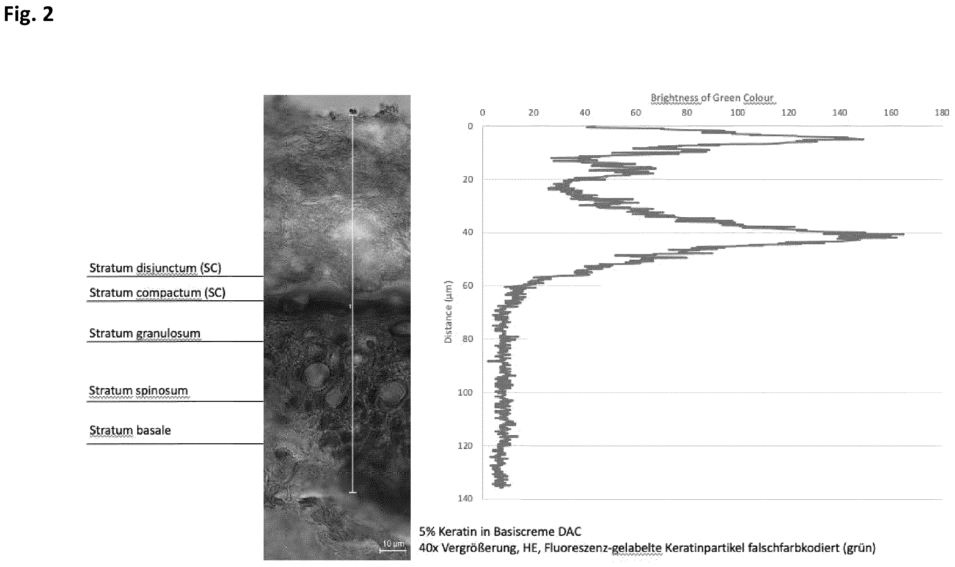

In Fig. 2 sind Querschnitte der oberen Hautschichten, auf die eine Formulierung mit 5 % kolloidaler Keratin-Lösung in DAC-Basiscreme und 1 % Phenoxyethanol aufgetragen wurde, gezeigt. Die Keratinpartikel wurden mit Isatosäureanhydrid fluoreszenzmarkiert. Die Abbildung ist ein Overlay aus einem Nativbild und einem Fluoreszenzbild (aufgenommen mit DAPI-Filter (blau) und anschließend falschfarbcodiert in grün). Es wird deutlich, dass die Keratinpartikel keine relevante Tiefenpenetration zeigen, sondern sich insbesondere in den oberen, lockeren Stratum corneum-Schichten (Stratum disjunctum) anreichern.

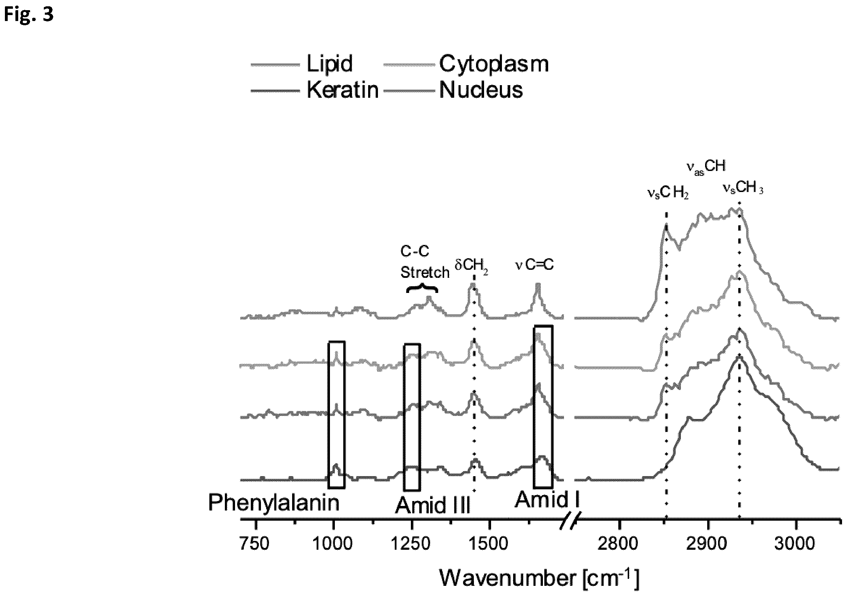

In Fig. 3 sind Raman-Spektren im Bereich der Wellenzahl von 750 cm⁻¹ bis 3000 cm⁻¹ für die Lipide (rote Linie), das Zytoplasma (grüne Linie), die Nuclei (blaue Linie) und die kolloidale Keratin-Lösung (graue Linie) dargestellt. Die Detektion der Raman-Spektren durch die laserbedingte Anregung der Elemente erfolgte im Wellenlängenbereich von 532 nm. Die Spektren für die Lipide, das Zytoplasma und die Nuclei konnten in den mit 500 µg kolloidaler Keratin-Lösung behandelten HaCaT-Zellen ermittelt werden. Das Spektrum der kolloidalen Keratin-Lösung wurde separat aufgenommen. Daraus lässt sich ableiten, dass die Keratinpartikel zwar an der Zelloberfläche über nicht-kovalente Interaktionen adhärieren, aber nicht von den Zellen aufgenommen werden.

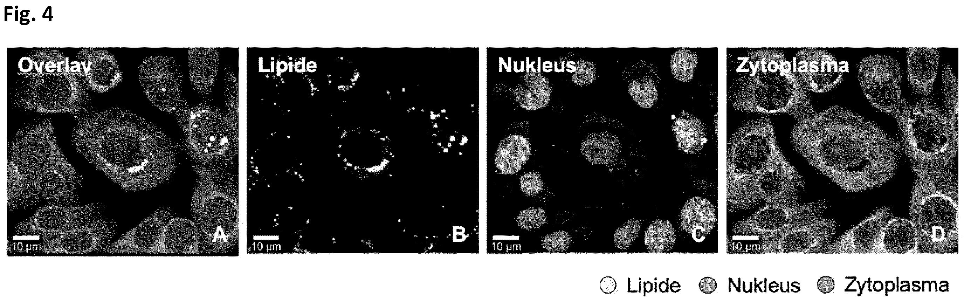

Fig. 4 zeigt ein Monitoring der Raman-Spektren für die mit 500 µg kolloidaler Keratin-Lösung behandelten HaCaT-Zellen. Die Detektion der Raman-Spektren durch die laserbedingte Anregung der Elemente erfolgte bei einer Wellenlänge von 532 nm. Die Spektren für die Lipide, die Nuclei und das Zytoplasma der HaCaT-Zellen wurden in allen drei Dimensionen aufgenommen und im Overlay, gekennzeichnet durch die Farben Gelb, Blau und Grün, zusammengelagert. Daraus lässt sich ableiten, dass die untersuchten Zellen keinen Uptake der Keratinpartikel zeigen.

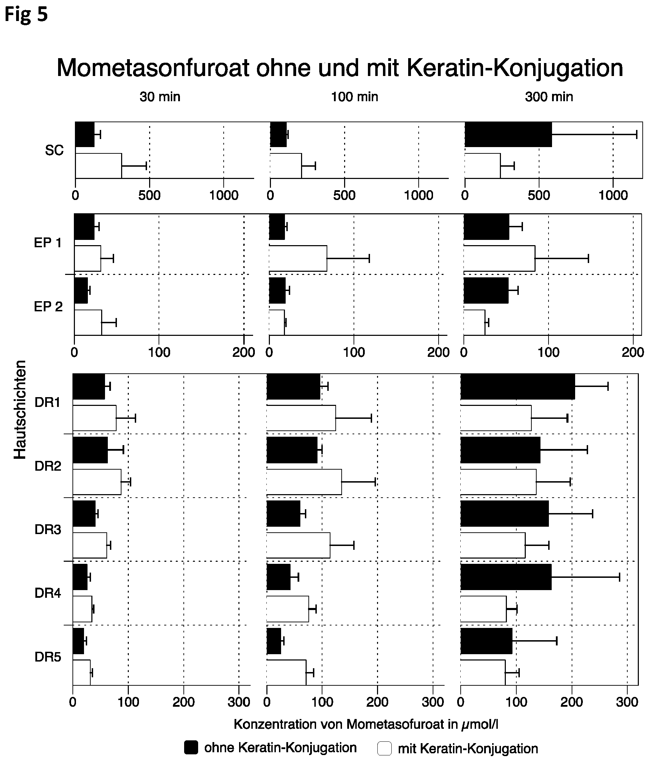

In Fig. 5 ist ein Konzentrations-Zeit-Profil von Mometasonfuroat (in µmol/l) in ex vivo Humanhaut nach 30, 100 und 300 min Applikationsdauer ohne und mit Keratin-Konjugation dargestellt. Die Untersuchungen wurden im Franz-Diffusionsmodell durchgeführt. Die Analyse der Mometasonfuroat-Konzentration erfolgte mittels HPLC. Die Daten zeigen, dass durch die Bildung von Keratin-Arzneistoff-Konjugate die Pharmakokinetik des Arzneistoffs relevant beeinflusst wird. So stellt sich eine erhöhte Freisetzungsrate des Konjugates und eine beschleunigte Diffusion durch ein höheres Konzentrationsgefälle des Arzneistoffs dar.

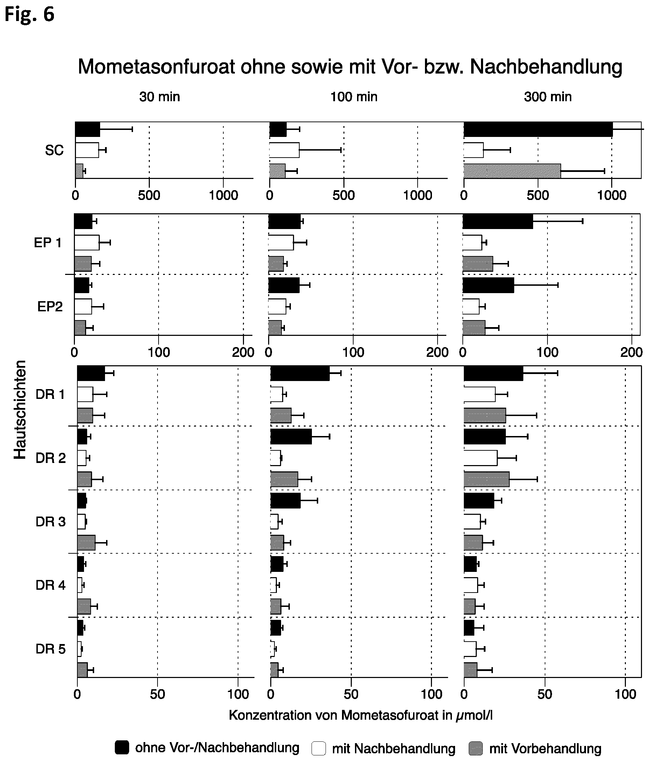

In Fig. 6 ist ein Konzentrations-Zeit-Profil von Mometasonfuroat (in µmol/l) ohne Keratin-Konjugation in ex vivo Humanhaut nach 30, 100 und 300 min Applikationsdauer im Vergleich zu Vor- und Nachbehandlung mit einer Keratin-haltigen Formulierung dargestellt. Die Untersuchungen wurden im Franz-Diffusionsmodell durchgeführt. Die Analyse der Mometasonfuroat-Konzentration erfolgte mittels HPLC. Die Daten zeigen, dass die Vor- bzw. Nachbehandlung mit einer Keratinpartikel-enthaltenen halbfesten Zubereitung keinen relevanten Einfluss auf die kutane Bioverfügbarkeit des Modelarzneistoffs hat. Die weist darauf hin, dass keine nicht-kovalente Konjugatbildung zwischen Keratin und Arzneistoff vorliegt.

### Beispiel 1

Als Keratinquelle wurden verschiedene tierische Materialien ausgewählt, die aber aufgrund ihres Proteinvernetzungsgrades unterschiedlich geeignet erscheinen. Nach Voruntersuchungen mit unterschiedlichen Keratin-enthaltenden Biomaterialien zeigte sich eine besondere Eignung von Vogelfedern. Neben praktischen Aspekten der Verfügbarkeit und einfacher Verarbeitung wurden vor allem biochemische Aspekte erarbeitet, die eine präferenzielle Verwendung von Federn begründen. Das in Federn enthaltene Keratin entspricht vorwiegend β-Keratin, welches als Polypeptidkette mit einer beta-Faltblattstruktur ausgestattet ist, aus Filamenten von 3-4 nm besteht und eine molekulare Masse von ca. 10-22 kDa aufweist. Im Unterschied zu α-Keratin bei Säugetieren finden sich bei beta-Keratin wenige, aber funktionell bedeutende Unterschiede in der Primärsequenz. So bildet beta-Keratin weniger Makrofibrillen als α-Keratin und zeigt ein regelmäßigeres Ordnungs- und Packungsverhalten. Diese Unterschiede bieten somit günstigere Voraussetzungen für eine Standardisierung der Keratinisolierung und der daraus resultierenden Produkteigenschaften. Dabei gilt es insbesondere die häufig praktizierte Hydrolysierung von Keratin zu vermeiden, um intakte Keratinproteine zu isolieren und die Bildung von Keratinbruchstücken, Aminosäuren und Peptide gezielt zu vermeiden. Für eine effektive und standardisierte Keratinisolierung wurde, basierend auf den biochemischen Besonderheiten von beta-Keratin, ein gesondertes Verfahren entwickelt. Die verwendeten Hühnerfedern wurden zunächst mit Wasser und Seife gereinigt, mit 70%-igem Ethanol desinfiziert und anschließend bei Raumtemperatur getrocknet. Nach der Zerkleinerung der gesäuberten und getrockneten Federn in einer Schneidmühle (Typ Retsch SM 100 comfort) erfolgte eine Homogenisierung des gesamten Materials. Mittels einer Soxhlet-Apparatur wurde das Federhomogenat entfettet. Für die Extraktion des Keratins wurde das entfettete Federmaterial zu einem Extraktionspuffers gegeben und 48 h lang extrahiert. Anschließend wurde der Extrakt zentrifugiert und das Sediment verworfen. Der erhaltene reine Extrakt wurde mit Wasser verdünnt und einer Filtration mit einem Cut off von 10.000 NMWC unterzogen. Mittels Sprühtrocknung wurde das Dialysat zu einem Pulver mit einer Partikelgröße von < 25 µm verarbeitet.

Zur Bestimmung der kolloidalen Größe der Keratinpartikel (1 mg/ml deionisiertes Wasser) wurden Messungen per dynamischer Lichtstreuung (DLS) mittels dem Gerät Zetasizer ZEN3600 von Malvern Panalytical Instruments nach DIN ISO 22412:2018-09 durchgeführt. Die Analysemethode ermöglicht die Charakterisierung von Partikelgrößen in Suspensionen und Emulsionen, bei der das emeritierte Streulicht eines Lasers ermittelt wird. Ziel war die Erfassung der Partikelgröße des Proteins im kolloidalen Zustand. Für die Messung der DLS wurde 1 ml einer 0,1 mM kolloidalen Keratin-Lösung in eine Einmalküvette aus Polystyrol pipettiert und anschließend in das auf 25 °C temperierte Küvetten-Modul des Zetasizer Lab überführt. Die Partikelgröße wurde mit Hilfe des automatischen Analysemodus (General purpose) und rückwärtigen Lichtstreuung in einem Winkel von 173° ermittelt. Jede Messung erfolgte als Triplikat und umfasste jeweils 15 Messzyklen pro Durchlauf. Die erfassten Messdaten pro Probe wurden anschließend gemittelt. Insgesamt wurden drei Proben mittels DLS untersucht. Im Rahmen der DLS wurde der Polydispersitätsindex (Pdl), welcher angibt wie homogen die Partikel innerhalb der Probe verteilt sind, die Größe der Partikel (in nm) und der prozentuale Anteil derjeweiligen Partikelgröße in Relation zum gesamten Probeninhalt, bestimmt.

Zur Bestimmung der Molekulargröße (Molekulargewicht) der extrahierten Keratinpartikel wurde eine Natriumdodecylsulfat-Polyacrylamidgelelektrophorese (SDS-PAGE) in einer SDS-Gelkammer (Mini Gel Tank von Invitrogen) durchgeführt. Die Proben wurden nach dem Herstellerprotokoll NuPAGE^{®} Bis-Tris Mini Gel Electrophoresis (Thermo Fisher) denaturiert. Hierfür wurden 2 µl Probe, 2,5 µl NuPAGE^{®} LSD Sample Buffer (4x), 1 µl NuPAGE^{®} LSD Reducing Agent (10x) und 6,5 µl DI-Wasser zusammengegeben und für 10 min bei 95 °C inkubiert. Zur Durchführung der Gelelektrophorese wurde die Gelkammer mit einem 1 × Tris-Tricin-Laufpuffer (1,2 M Tris, 0,8 M Tricin, 2 % SDS) gefüllt. Anschließend wurde das Tris-Tricin-Gel (Novex 10-20 % Tricin Gele von Invitrogen (Thermo Fisher Scien-tific), LOT 20101945, REF EC6625BOX) eingesetzt und der Kamm aus dem Gel entfernt. Je 10 µl der vorbereiteten Proben und jeweils 2 µl des Markers wurden in die entsprechenden Geltaschen gegeben. Die Gelelektrophorese wurde bei 130 V und 250 mA für 1,5 Stunden durchgeführt. Das Gel wurde anschließend aus der Kammer entfernt und der Laufpuffer verworfen. Die Plastik-Gel-Halterung wurde aufgebrochen und das Tris-Tricin-Gel in einen Behälter mit Coomassie-Brilliant-Blue G250 Färbemittel (2 ml 5 % Coomassie-Lösung, 3 ml Ortho-Phosphatsäure, 20 ml Ethanol, 10 g Ammoniumsulfat, 65 ml dH2O) überführt. Das Gel wurde in der Lösung über Nacht auf dem Schüttler inkubiert. Im Anschluss wurde die Färbelösung entfernt. Zur Entfärbung wurde das Gel mehrmals mit destilliertem Wasser, unter leichtem Schütteln, gespült.

Es wurde versucht, die Funktionalität eines Korneozyten nachzustellen. Dazu wurde aus verschiedenen natürlichen Keratinquellen Keratin extrahiert und entsprechende Partikel generiert, die zunächst als mechanische Stabilisierung von Lipidmatrices in verschiedenen halbfesten Zubereitungen untersucht wurden. Dabei war beabsichtigt, die Keratinpartikel mit bipolaren Lipiden zu ummanteln, um Keratosomen zu entwickeln.

Zunächst wurden verschiedene Extraktionsmethoden an humanem Haar (alpha-Keratin) getestet. Überwiegend wurde die chemische Denaturierung zur Zersetzung des Haares genutzt. Dafür wurden Urea, Thiourea und Guanidiuniumhydrochlorid eingesetzt. Zur Unterstützung der Denaturierung fanden als Reduktionsmittel β-Mercaptoethanol, Cysteamine, L-Cystein Verwendung. Höchste Ausbeuten wurden mit einer Extraktionslösung aus 5 M Guanidiuniumhydrochlorid, 10 % Cysteamine, 25 mM Tris bei pH 8,5 erzielt. Auf Grund der Unbedenklichkeit der Substanzen Urea und L-Cystein wurde zur Isolierung des Keratins eine Extraktionslösung bestehend aus 10 M Urea, 100 mM L-Cysteine, 25 mM Tris-HCl bei pH 10,5 ausgewählt. Nach erfolgreicher Extraktion wurde die Extraktionslösung dialysiert (cutoff: 6-8 kDa, regenerierte Cellulose, SpectraPor^{®}), dabei entsteht eine kolloidale Lösung, bei hoher Proteinkonzentration und großen Dialyseschritten (5-6 I) fällt Keratin im Dialyseschlauch aus. Final wurde das Dialysat lyophilisiert, wodurch ein weißes Pulver entsteht. Dieses Pulver wurde mittels Rasterelektronenmikroskopie (REM) untersucht. Die Partikel aus Haarkeratin weisen eine variierende Größe und Form auf. Dabei wurde definiert, dass die Partikel in der finalen Formulierung eine Größe von >600 nm aufweisen sollten, um eine Penetration durch die Hornschicht der Haut zu vermeiden.

Aus regulatorischen Gründen wurde als alternative Keratinquelle Federkeratin (beta-Keratin) von verschiedenen Vogelarten verwendet. Hierzu wurden Rohfedern von Hühnern, Gänsen und Enten untersucht. Aus praktischen Erwägungen wurden insbesondere Hühnerfedern für die weiteren Untersuchungen eingesetzt.

Deshalb wurde die Extraktion von Federkeratin an den etablierten Extraktionsprozess für Haarkeratin angepasst. Dabei wurde beobachtet, dass im Vergleich zu allen anderen untersuchten Extraktionsverfahren mit der ausgewählten Extraktionslösung bestehend aus 10 M Urea, 100 mM L-Cystein, 25 mM Tris-HCl bei pH 10,5 die höchste Ausbeute zu erzielen ist. Die optionale Zugabe vom 1 M Ammoniumchlorid, verhindert die Carbamylierung am Protein, und die damit einhergehenden Veränderungen der Proteineigenschaften.

Zur Größenbestimmung wurden die Feder- und Haarkeratinpartikel vergleichend mittels Transmissionselektronenmikroskopie (TEM) im negativ Stain untersucht. Haarkeratinpartikel in kolloidaler Lösung wiesen eine Größe von ca. 40-75 nm, hingegen für die Federkeratinpartikel eine Größe von ca. 20-35 nm auf. Zur Visualisierung der Keratinpartikel für die Penetrationsuntersuchung wurden diese mit 2-Aminobenzoyl (Abz) fluoreszenz-markiert (Reaktion mit Isatosäureanhydrid unter basischen, denaturierenden Bedingungen). 5 % (w/w) fluoreszenzmarkiertes Haar- und Federkeratin wurde in Basiscreme DAC formuliert und nach Objektträgerausstrich fluoreszenzmikroskopisch untersucht. Die einzelnen Partikel aus den kolloidalen Lösungen, welche mittels TEM identifiziert wurden, bilden in Basiscreme DAC anteilig Aggregate. Für Federkeratin ist eine homogene Verteilung der zusammengelagerten Partikel zu erkennen, die Partikelgrößen liegen zwischen 20 und 35 µm. Haarkeratin hingegen lagert sich zu größeren, kristallartigen Partikeln zusammen. Die Partikelgrößen liegen hier zwischen 20 und 140 µm.

Mittels Penetrationsuntersuchungen von Haar- und Federkeratin (jeweils 5% (w/w)) an *ex vivo* Humanhaut (Franzzelle) in Basiscreme DAC konnte zudem nachgewiesen werden, dass die Keratinpartikel nicht in tiefe Hautschichten diffundieren, sondern in den oberen Anteilen des SC (Stratum disjunctum) verbleiben. Um die Interaktion der Federkeratinpartikel mit den SC-Lipiden bzw. Effekte von Lipidbeschichtungen besser einschätzen zu können, wurde bei verschiedenen pH-Werten das Zeta-Potential bestimmt (Tab. 1).

**Tab. 1: Zeta-Potential von Federkeratin und fluoreszenz-markiertem Federkeratin jeweils 10mg/ml in 10mM Kaliumchlorid bei verschiedenen pH-Werten. Messung durchgeführt von Fabio.**

| **pH-Wert** | **Federkreatin 10 mg/mL** | **Federkreatin-Abz** |
|---|---|---|
| 10,5 | -25 mV | -24,2 mV |
| 7,6 | -1,5 mV | -23,3 mV |
| | Versuch 1: -17,8 mV | |
| 5,5 | -20,2 mV | -23,3 mV |

Die Keratinpartikel weisen eine negative Ladung auf, was sie zur Beschichtung mit positiv-geladenen Lipiden prädestiniert.

### Beispiel 2

### Zytotoxikologische Untersuchungen

Die Zelltoxizität der Keratinpartikel wurde mittels der Zellproliferationsrate, anhand der Bromdesoxyuridin (BrdU) Einlagerung während der DNA-Synthese der Zellen nach 24- und 48-stündiger Keratinpartikel-Behandlung bestimmt. Um die Vitalität der Zellen nach erfolgtem Keratinpartikel-Zusatz zu ermitteln, wurde die Anzahl der lebensfähigen Zellen basierend auf der Quantifizierung der ATP-Präsens, nachgewiesen. In beiden Untersuchungsansätzen wurden die Keratinpartikel als kolloidale Lösung in unterschiedlichen Konzentrationen getestet, um die Zell-Keratinpartikel-Interaktion zu bewerten.

Zur Herstellung einer kolloidalen Keratin-Lösung wurden 142,5 mg Keratinpartikel in 5 ml Denaturierungspuffer gelöst. Anschließend wurde das Lysat in eine Dialysemembran überführt und gegen DI-Wasser, im Austausch 1:200, dialysiert unter Erhalt einer Endkonzentration von 21,9 mg/ml. Die Lösung wurde bis zur Verwendung bei 4 °C gelagert. Für die Durchführung der Untersuchungen wurde die kolloidale Keratinpartikel-Lösung (c = 10 mg/ml) mit DI-Wasser verdünnt.

Für die Zellproliferations- und Zellvitalitätstests wurden native humane dermale Fibroblasten (NHDF) und native humane epitheliale Keratinozyten (NHEK) in das Fibroblasten-Medium und SFM-Keratinozytenmedium aufgenommen. Danach wurde die Zellzahl jeder Zellsuspension mittels Fuchs-Rosenthal-Zählkammer bestimmt. Anschließend konnte die Zellsuspension jeder Zelllinie mit ihrem entsprechenden Wachstumsmedium zu einer Konzentration von 0,003 Millionen Zellen verdünnt werden. In jeweils sechs Wells einer 96-Well-Platten wurden pro Versuch jeweils 100 µl der eingestellten Zellsuspension pipettiert. Insgesamt wurden für jeden Test und Untersuchungszeitpunkt (24 h oder 48 h) drei Ansätze angefertigt. Im Anschluss wurden die NHDF-Zellen für 72 Stunden und die NHEK-Zellen für 96 Stunden bei 37°C und 5% CO₂ kultiviert. Um den Einfluss der Keratinpartikel auf die Proliferation und Vitalität der Zellen zu testen, wurde die kolloidale Lösung der Keratinpartikel in entsprechenden Endkonzentrationen von 0,1%, 0,05%, 0,01%, 0,005% und 0,001% in jeweils einem Well pro Ansatz zugesetzt. Die eingesäten Zellen mit Keratinpartikel-Zusatz wurden anschließend für 24 Stunden bzw. 48 Stunden unter den genannten Bedingungen kultiviert, bevor das Medium mit enthaltener Keratinkonzentration wieder abgesaugt wurde. Danach wurden die Zellen mit frischem Medium gespült und für die Durchführung der Tests 100 µl frisches Zellmedium zugesetzt.

Zur Überprüfung der Zellproliferation der mit Keratinpartikel behandelten Zellen wurde ein BrdU-Assay durchgeführt. Dafür wurde in jedes mit Zellen kultivierte Well 10 µl des BrdU Labeling Reagents gegeben und verteilt. Die Platten wurden anschließend für durchschnittlich drei Stunden bei 37°C und 5% CO₂ kultiviert. Nach der Inkubationszeit wurde das BrdU Labeling Reagent abgesaugt und pro Well 200 µl FixDent dazugegeben und für 30 Minuten bei Raumtemperatur inkubiert. Das FixDent wurde im Anschluss wieder abgesaugt und die überschüssige Lösung durch das Ausklopfen auf ein Tuch entfernt. Daraufhin konnten 100 µl Anti-BrdU-Peroxidase-Arbeitslösung in jedes Well gegeben und für weitere 90 Minuten bei Raumtemperatur inkubiert. Die Anti-BrdU-Peroxidase-Arbeitslösung wurde danach wieder abgenommen und jedes Well dreimal mit jeweils 200 µl Waschlösung gewaschen. Um die restliche Lösung zu entfernen, wurde die Platte auf einem Tuch ausgeklopft. Schließlich konnten pro Well 100 µl Substrate Solution TMB zugegeben und für ca. 30 Minuten im Dunkeln bei Raumtemperatur inkubiert werden. Die Absorption der BrdU-Bindung in den Zellen konnte danach bei einer Wellenlänge von 370 nm gemessen werden.

Die ATP-Präsens und damit das Vitalitätsniveaus der Zellen wurde mittels Cel-ITiter-Glo Assay bestimmt. Dazu wurden zu je 100 µl Zellmedium mit enthaltenen Zellen pro Well, 100 µl des Nachweisreagenzes gegeben und anschließend für zwei Minuten geschüttelt. Nach dem Induzieren der Zelllyse wurde die Platte für zehn Minuten bei Raumtemperatur inkubiert, um das Signal für die folgende Lumineszenzmessung zu stabilisieren. Im letzten Schritt wurde das Lumineszenzsignal pro Well und damit pro applizierter Keratinpartikel-Konzentration ermittelt. Der Anteil der ATP-Präsens pro Well wurde mittels einer zuvor erstellten ATP-Standard-Kurve bestimmt.

Die Proliferationsrate und das Vitalitätsniveau der NHDF- und NHEK-Zellen wurde nach 24- sowie 48-stündiger Inkubation unter verschiedenen Konzentrationen der kolloidalen Keratin-Lösung bestimmt, um einen möglichen toxischen Einfluss der Keratinpartikel auszuschließen.

Zum Nachweis der Proliferationsrate wurde ein BrdU-Assay durchgeführt, bei welchem die BrdU-Einlagerung während der DNA-Synthese der Zellen bestimmt wurde. Für die NHDF-Zellen konnte bei der Zugabe der Keratinpartikel keine Verringerung der Zellproliferation, weder nach 24- noch 48-stündiger Inkubation, nachgewiesen werden. Im Gegensatz dazu konnte eine Reduktion der Proliferationsrate der NHEK-Zellen nach der Applikation der kolloidalen Keratin-Lösung verzeichnet werden. Ab einer Konzentration von 0,05 % wurde eine leichte Abnahme der Zellteilung nach 24- stündiger Inkubation mit den Keratinpartikeln ermittelt. Im Zuge der 48-stündigen Behandlung der NHEK-Zellen wurde eine deutliche Abnahme der Zellproliferationsrate ab einer Konzentration von 0,005 % bestimmt. Diese ist vermutlich auf das hygroskopische und leicht quellende Verhalten der Keratinpartikel zurückzuführen. Während des Inkubationsschrittes quellen die Keratinpartikel auf und legen sich wie ein Film über die Zellen, der auch durch den Medienwechsel nicht abgenommen werden konnte. Aufgrund der Filmbildung konnte nicht ausgeschlossen werden, dass die Zellen bei dem verwendeten Versuchsdesign an ihrer Zellatmung gehindert wurden und somit die Zellproliferationsrate abnahm. Da dieses Phänomen nicht bei den ebenso getesteten NHDF-Zellen beobachtet werden konnte, kann ein Einfluss der Keratinpartikel auf das Proliferationsverhalten der NHEK-Zellen weitestgehend ausgeschlossen werden. Vielmehr ist davon auszugehen, dass die reduzierte Proliferation der NHEK-Zellen sich auf ein beeinträchtigendes Zusammenspiel zwischen Zellmedium und Keratinpartikel begründet, dass sich negativ auf die Einsatzkomponenten des BrdU-Assays auswirkte.

Im Rahmen des CellTiter Glo-Assays wurde die Vitalität der Zellen anhand der ATP-Präsens bestimmt. Wie aus Tabelle 8 hervorgeht, veränderte sich die Vitalität der NHDF-Zellen nicht durch eine 24- oder 48-stündige Behandlung mit den Keratinpartikeln. Der Einsatz der Keratinpartikel bis zu einer Konzentration von 0,1% hat demnach keinen Einfluss auf die Vitalität der NHDF-Zellen, sowohl nach 24- als auch 48-stündiger Inkubation. Bei der Bestimmung der ATP-Präsens in den NHEK-Zellen konnte hingegen eine starke Abnahme der Zellvitalität nach erfolgter Applikation der Keratinpartikel verzeichnet werden. Bereits nach 24 Stunden wurde bei einer Einsatzkonzentration von 0,05 % eine Vitalitätsabnahme der NHEK-Zellen von rund 20% beobachtet. Nach der 48-stündigen Inkubation der Zellen mit den Keratinpartikeln konnte eine verringerte Zellvitalität bei jeder der eingesetzten Konzentrationen (von 0,001 % bis 0,1 %) nachgewiesen werden. Besonders bei den Einsatzkonzentrationen von 0,05 % und 0,1 % konnte nur noch ein ATP-Präsens von ca. 40 % und 30 % bestimmt werden. Im Verlauf der Untersuchungen konnte hier jedoch beobachtet werden, dass sich die kolloidale Keratinlösung auf den NHEK-Zellen filmartig ablagerte und sich auch nach dem Medienwechsel nicht entfernen ließ. Hier könnten die Zellen an ihrer Atmung gehindert worden sein, was zum Absterben der Zellen geführt haben könnte. Weiterhin wäre es möglich, wie bereits für den BrdU-Assay beschrieben, dass ein nachteiliges Zusammenspiel von Zellmedium und den kolloidalen Keratinpartikel vorlag. Ein toxischer Effekt der kolloidalen Keratinpartikel kann ausgeschlossen werden, da die NHDF-Zellen keine Beeinträchtigung in ihrer Zellvitalität aufzeigten. Es wird davon ausgegangen, dass die auf der Zelloberfläche abgelagerten Keratinpartikel die Nachweismittel an der Bestimmung der Zellvitalität beeinträchtigten.

**Tab. 2: Bestimmung der Zellproliferationsraten der NHDF-Zellen mittels BrdU-Assay nach Applikation der Keratinpartikel für 24 h und 48 h. Keratin wurde als kolloidale Lösung in einer Endkonzentration von 0,001 %, 0,005 %, 0,01 %, 0,05 % und 0,1 % appliziert. (M-KO=Zellmediumskontrolle, Lsg.-KO=Lösungsmittelkontrolle, MW-KO=Mittelwert der Zellmediumskontrolle, IndexKO=Berechnung der Zellproliferationsrate).**

| **Applikation der kolloidalen Keratin-Lösung an NHDF-Zellen** | | | | | | | | | | | | | | |
|---|---|---|---|---|---|---|---|---|---|---|---|---|---|---|
| | 24 h | | | | | | | 48 h | | | | | | |
| | M-KO | Lsg.-KO | 0,001 % | 0,005 % | 0,01 % | 0,05 % | 0,1% | M-KO | Lsg.-KO | 0,001 % | 0,005 % | 0,01 % | 0,05 % | 0,1% |
| | 2,0427 | 2,5108 | 2,2787 | 2,0767 | 2,5743 | 1,4139 | 2,3664 | 1,6191 | 1,8711 | 1,9595 | 2,1022 | 2,2412 | 2,0773 | 2,1937 |
| | 1,9631 | 2,1754 | 2,2628 | 2,2351 | 1,8600 | 2,4720 | 2,3863 | 1,7020 | 1,8196 | 2,0636 | 2,2881 | 2,4194 | 2,1372 | 2,3055 |
| | 2,4367 | 2,3371 | 2,6008 | 2,4288 | 2,5175 | 2,5200 | 2,5026 | 1,8011 | 1,7522 | 2,1139 | 2,3166 | 2,1548 | 1,9297 | 2,2334 |
| Berechnung MW-KO | 2,15 | | | | | | | 1,71 | | | | | | |
| IndexKO | 0,95 | 1,23 | 1,12 | 1,02 | 1,26 | 0,69 | 1,16 | 0,95 | 1,16 | 1,21 | 1,30 | 1,38 | 1,28 | 1,35 |
| | 0,91 | 1,11 | 1,15 | 1,14 | 0,95 | 1,26 | 1,22 | 1,00 | 1,07 | 1,21 | 1,34 | 1,42 | 1,26 | 1,35 |
| | 1,13 | 0,96 | 1,07 | 1,00 | 1,03 | 1,03 | 1,03 | 1,05 | 0,97 | 1,17 | 1,29 | 1,20 | 1,07 | 1,24 |
| MW | 1,00 | 1,10 | 1,11 | 1,05 | 1,08 | 1,00 | 1,13 | 1,00 | 1,07 | 1,20 | 1,31 | 1,33 | 1,20 | 1,32 |

**Tab. 3: Bestimmung der Zellproliferationsraten der NHEK-Zellen mittels BrdU-Assay nach Applikation der Keratinpartikel für 24 h und 48 h. Keratin wurde als kolloidale Lösung in einer Endkonzentration von 0,001 %, 0,005 %, 0,01 %, 0,05 % und 0,1 % appliziert. (M-KO=Zellmediumskontrolle, Lsg.-KO=Lösungsmittelkontrolle, MW-KO=Mittelwert der Zellmediumskontrolle, IndexKO=Berechnung der Zellproliferationsrate).**

| **Applikation der kolloidalen Keratin-Lösung an NHEK-Zellen** | | | | | | | | | | | | | | |
|---|---|---|---|---|---|---|---|---|---|---|---|---|---|---|
| | 24 h | | | | | | | 48 h | | | | | | |
| | M-KO | Lsg.-KO | 0,001 % | 0,005 % | 0,01 % | 0,05 % | 0,1% | M-KO | Lsg.-KO | 0,001 % | 0,005 % | 0,01 % | 0,05 % | 0,1% |
| | 1.3447 | 1.2008 | 1.5210 | 1.2439 | 1.2264 | 1.0608 | 1.0313 | 2.2751 | 2.2606 | 2.0016 | 1.4958 | 1.2353 | 0.7959 | 1.0016 |
| | 1.2038 | 1.0383 | 1.2802 | 1.2797 | 1.2693 | 0.9879 | 0.9967 | 1.9802 | 1.8486 | 1.8772 | 1.3192 | 1.0617 | 0.8870 | 0.9714 |
| | 1.8927 | 1.8776 | 1.6588 | 1.8287 | 1.8413 | 1.8121 | 1.8508 | 2.2163 | 1.9189 | 1.9900 | 2.0637 | 1.9630 | 2.1360 | 1.9085 |
| Berechnung MW-KO | 1.48 | | | | | | | 2.16 | | | | | | |
| IndexKO | 0.91 | 0.89 | 1.13 | 0.93 | 0.91 | 0.79 | 0.77 | 1.05 | 0.99 | 0.88 | 0.66 | 0.54 | 0.35 | 0.44 |
| | 0.81 | 0.86 | 1.06 | 1.06 | 1.05 | 0.82 | 0.83 | 0.92 | 0.93 | 0.95 | 0.67 | 0.54 | 0.45 | 0.49 |
| | 1.28 | 0.99 | 0.88 | 0.97 | 0.97 | 0.96 | 0.98 | 1.03 | 0.87 | 0.90 | 0.93 | 0.89 | 0.96 | 0.86 |
| MW | 1,00 | 0,92 | 1,02 | 0,98 | 0,98 | 0,86 | 0,86 | 1,00 | 0,93 | 0,91 | 0,75 | 0,65 | 0,59 | 0,60 |

**Tab. 4: Bestimmung der Zellvitalitätsrate der NHDF-Zellen mittels CellTiter Glo-Assay nach KeraBods-Applikation für 24 h und 48 h. Keratin wurde als kolloidale Lösung in einer Endkonzentration von 0,001 %, 0,005 %, 0,01 %, 0,05 % und 0,1 % appliziert. (M-KO=Zellmediumskontrolle, Lsg.-KO=Lösungsmittelkontrolle, MW-KO=Mittelwert der Zellmediumskontrolle, IndexKO=Berechnung der Zellproliferationsrate).**

| **Applikation der kolloidalen Keratin-Lösung an NHDF-Zellen** | | | | | | | | | | | | | | |
|---|---|---|---|---|---|---|---|---|---|---|---|---|---|---|
| | 24 h | | | | | | | 48 h | | | | | | |
| | M-KO | Lsg.-KO | 0,001 % | 0,005 % | 0,01 % | 0,05 % | 0,1% | M-KO | Lsg.-KO | 0,001 % | 0,005 % | 0,01 % | 0,05 % | 0,1% |
| | 156380 | 179857 | 185967 | 189359 | 181053 | 193909 | 170696 | 194354 | 237065 | 234680 | 225535 | 210292 | 210010 | 202445 |
| | 143590 | 160705 | 166304 | 163818 | 159262 | 162065 | 153999 | 193855 | 215134 | 209728 | 195124 | 183936 | 176625 | 182405 |
| | 241802 | 241880 | 242830 | 234451 | 238568 | 228865 | 209243 | 214392 | 209133 | 228103 | 218148 | 227969 | 224300 | 217663 |
| Berechnung MW-KO | 180591 | | | | | | | 200867 | | | | | | |
| IndexKO | 0,87 | 1,15 | 1,19 | 1,21 | 1,16 | 1,24 | 1,09 | 0,97 | 1,22 | 1,21 | 1,16 | 1,08 | 1,08 | 1,04 |
| | 0,80 | 1,12 | 1,16 | 1,14 | 1,11 | 1,13 | 1,07 | 0,97 | 1,11 | 1,08 | 1,01 | 0,95 | 0,91 | 0,94 |
| | 1,34 | 1,00 | 1,00 | 0,97 | 0,99 | 0,95 | 0,87 | 1,07 | 0,98 | 1,06 | 1,02 | 1,06 | 1,05 | 1,02 |
| MW | 1,00 | 1,09 | 1,12 | 1,11 | 1,08 | 1,11 | 1,01 | 1,00 | 1,10 | 1,12 | 1,06 | 1,03 | 1,01 | 1,00 |

**Tab. 5: Bestimmung der Zellvitalitätsrate der NHEK-Zellen mittels CellTiter Glo-Assay nach KeraBods-Applikation für 24 h und 48 h. KeraBods wurden in kolloidaler Lösung und als Feststoff in einer Endkonzentration von 0,001 %, 0,005 %, 0,01 %, 0,05 % und 0,1 % appliziert. (M-KO=Zellmediumskontrolle, Lsg.-KO=Lösungsmittelkontrolle, MW-KO=Mittelwert der Zellmediumskontrolle, IndexKO=Berechnung der Zellproliferationsrate).**

| **Applikation der kolloidalen Keratin-Lösung an NHEK-Zellen** | | | | | | | | | | | | | | |
|---|---|---|---|---|---|---|---|---|---|---|---|---|---|---|
| | 24 h | | | | | | | 48 h | | | | | | |
| | M-KO | Lsg.-KO | 0,001 % | 0,005 % | 0,01 % | 0,05 % | 0,1% | M-KO | Lsg.-KO | 0,001 % | 0,005 % | 0,01 % | 0,05 % | 0,1% |
| | 103591 | 120511 | 111548 | 120327 | 101526 | 90867 | 79794 | 191134 | 202193 | 193433 | 161420 | 122104 | 72962 | 42958 |
| | 103819 | 106339 | 101140 | 100219 | 92672 | 77332 | 67908 | 180029 | 175543 | 147776 | 154529 | 116512 | 78931 | 72361 |
| | 250895 | 232500 | 236673 | 209888 | 225615 | 201379 | 201382 | 310719 | 257100 | 241802 | 177204 | 183825 | 147780 | 114970 |
| Berechnung MW-KO | 152768 | | | | | | | 227294 | | | | | | |
| IndexKO | 0,68 | 1,16 | 1,08 | 1,16 | 0,98 | 0,88 | 0,77 | 0,84 | 1,06 | 1,01 | 0,84 | 0,64 | 0,38 | 0,22 |
| | 0,68 | 1,02 | 0,97 | 0,97 | 0,89 | 0,74 | 0,65 | 0,79 | 0,98 | 0,82 | 0,86 | 0,65 | 0,44 | 0,40 |
| | 1,64 | 0,93 | 0,94 | 0,84 | 0,90 | 0,80 | 0,80 | 1,37 | 0,83 | 0,78 | 0,57 | 0,59 | 0,48 | 0,37 |
| MW | 1,00 | 1,04 | 1,00 | 0,99 | 0,92 | 0,81 | 0,74 | 1,00 | 0,95 | 0,87 | 0,76 | 0,63 | 0,43 | 0,33 |

Zusammenfassend konnten keine beeinträchtigenden Eigenschaften der kolloidalen Keratinpartikel ermittelt werden, die zelltoxischen Ursprungs sind. Mittels des Versuchsdesigns wurde nachgewiesen, dass die NHDF- und NHEK-Zellen nach einer 24- als auch 48-stündigen Inkubation mit den Keratinpartikeln in den eingesetzten Konzentrationen keine Veränderungen in ihrer Proliferations- und Vitalitätsrate aufweisen.

### Scratch-Assay

Ein Scratch-Assay wurde durchgeführt, um die Wundheilungsfähigkeit der HaCaT- und NHDF-Zellen nach 2-, 4-, 8-, 12-, 24- und 48-stündiger Inkubation mit verschiedenen Keratinpartikel-Konzentrationen zu bestimmen.

Zur Durchführung des Scratch-Assays wurde eine künstliche Basalmembran in einer 6-Well Platte erzeugt. Dazu wurde unter sterilen Bedingungen je 1 ml des Laminin-Fibronektin-Kollagen IV-Gemischs (FKS) in je ein Well der Platte gegeben, über Nacht bei 4°C inkubiert und abgesaugt. Anschließend wurde in jedes Well 1 ml PBS-FKS gegeben und für 1 Stunde bei 37°C im Brutschrank inkubiert. Nach der Abnahme des PBS-FKS-Gemisches wurde jedes Well zweimal mit PBS gespült. Nach der vollständigen Entfernung der flüssigen Rückstände konnten auf die künstlichen Basalmembranen Zellen ausgesät werden.

Zur Durchführung des Scratch-Assays wurden die HaCaT- und NHDF-Zellen in das SFM-Keratinozytenmedium bzw. Fibroblasten-Medium aufgenommen. Danach wurde die Zellzahl jeder Zellsuspension mittels Fuchs-Rosenthal-Zählkammer bestimmt. Anschließend konnte die Zellsuspension jeder Zelllinie mit ihrem entsprechenden Wachstumsmedium zu einer Konzentration von 0,2 Millionen Zellen verdünnt werden. In jedes Well der 6-Well-Platte wurde 1 ml der eingestellten Zellsuspension pipettiert. Im Anschluss wurden die HaCaT-und NHDF-Zellen für 72 bis 96 Stunden bei 37°C und 5% CO₂ kultiviert. Nach 72 Stunden wurde die Konfluenz der Zellen unter dem Mikroskop überprüft und je nach Wachstumsstadium das Medium noch einmal erneuert. Um den Einfluss der Keratinpartikel auf die Wundheilungsfähigkeit der Zellen zu testen, wurden die kolloidalen Keratinpartikel in einer entsprechenden Endkonzentration von 0,1 %, 0,05 %, 0,01 %, 0,005 % und 0,001 % in jeweils ein Well pro Ansatz zugesetzt.

Nachdem eine ausreichende Konfluenz der Zellen erreicht und die entsprechende Keratinpartikel-Konzentration in die Wells pipettiert wurde, wurde als Ausgangspunkt ein Bild mit dem Mikroskop und der Software Cell R angefertigt. Unter sterilen Bedingungen wurde anschließend mit Hilfe einer 10 µl Pipettenspitze vertikal verlaufend eine gerade Linie eingekerbt, welche die Stelle der Läsion dargestellte. Im Anschluss daran wurde das Medium abgesaugt und die Platte zur Dokumentation verschlossen und umgedreht. Die Einwanderung der Zellen wurde so zu den Versuchszeitpunkten 0, 2, 4, 8, 12, 24 und 48 Stunden fotografisch dokumentiert. Anschließend wurden die Zellen erneut der entsprechenden Keratinpartikel-Konzentration bzw. dem Wachstumsmedium ausgesetzt. Zur Auswertung wurde die Läsionsfläche zu jedem Versuchszeitpunkt mittels der Software Cell R bestimmt.

Die Migrationsfähigkeit der HaCaT- und NHDF-Zellen (Wundheilungsfähigkeit) unter Keratinpartikel-Behandlung wurde im Rahmen des Scratch-Assays untersucht. Dafür wurde die reepithelisierte Fläche innerhalb von 48 Stunden dokumentiert. Nach 48 Stunden konnten bei der Kontrolle keine Läsionen mehr verzeichnet werden; die reepithalisierte Fläche beider Zelllinien entsprach 100 %. Im Gegensatz dazu konnte eine Abnahme der bewachsenen Fläche mit steigender Keratinpartikel-Konzentration, sowohl bei den HaCaT- als auch NHDF-Zellen nach 48-stündiger Behandlung mit den kolloidalen Keratinpartikeln, ermittelt werden. Dabei zeigten die HaCaT-Zellen eine deutlich geringere Migrationsfähigkeit als die NHDF-Zellen. Nach der Applikation der kolloidalen Partikel nahm die Migrationsfähigkeit der HaCaT-Zellen deutlich ab und befand sich bei einer Einsatzkonzentration von 0,001 % bereits unter 50 %. Mit steigendem Keratinpartikel-Gehalt (0,005% bis 0,1 % Keratin in Lösung) sank die Fähigkeit der Reepithelisierung der HaCaT-Zellen weiter ab und umfasste nur noch durchschnittlich 19 %. Im Gegensatz dazu zeigten die NHDF-Zellen insgesamt eine wesentlich geringere Abnahme der Migrationsfähigkeit. Bei einer Einsatzkonzentration von 0,001% konnten noch 90% der Läsionsfläche durch die NHDF-Zellen besiedelt werden. Die Migration der NHDF-Zellen nahm mit nur noch 70 % wiederbesiedelter Fläche weiter ab, bei einer Anwendung von 0,005 % Keratinpartikel. Schließlich lag die Rebesiedlungsfläche der NHDF-Zellen, bei einem Keratinpartikel-Gehalt von 0,01 % und höher, bei rund 55 %.

Wie Fig. 1 zeigt, wirkt sich der Einsatz der Keratinpartikel auf das Migrationsverhalten der HaCaT- und NHDF-Zellen aus, wobei im Rahmen der Untersuchungen beobachtet werden konnte, dass sich die Keratinpartikel filmartig über die Zellen legten und somit eine mechanische Zellwachstumsbarriere bilden. Durch die zur Dokumentation notwendige Entfernung und erneute Applikation der Keratinpartikel wurde dieser Effekt noch verstärkt, was in der Bewertung der vorliegenden Ergebnisse beachtet werden muss.

### Präklinische Verträglichkeit (HET-CAM)

Als Screening hinsichtlich der Hautverträglichkeit der Keratinpartikel wurde dieses in eine halbfeste Formulierung eingearbeitet und an der Chorion-Allantois-Membran von acht- bis neuntägigen *Gallus gallus domesticus* Embryonen (Rasse: New Hampshire) getestet.

Die Hautverträglichkeit der Keratinpartikel wurde an der Chorion-Allantois-Membran von acht- bis neuntägigen Hühnerembryonen getestet. Befruchtete Hühnereier wurden hierfür acht bis neun Tage lang bei 37°C und 55 % Luftfeuchtigkeit bebrütet. Alle 12 Stunden wurden die Eier gewendet, ausgenommen die letzten 24 Stunden vor Versuchsbeginn. Die Öffnung der Eier wurde an den nach oben gerichteten Eierpolen durchgeführt, wofür die Eierschalen kreisrund (Ø 1,5 cm) geöffnet wurden. Nach dem Abnehmen der Eischalen wurden die Eihäute mit einer 37°C warmen 0,9%-igen NaCl-Lösung befeuchtet. Anschließend wurden unter der Sterilbank die äußeren Eihäute mittels Schere und Pinzette entfernt und die Chorion-Allantois-Membranen freigelegt. Etwa 10-30 % der präparierten Eier wurden für die Untersuchungen nicht verwendet, da sie unbefruchtet waren. Die halbfeste Keratinpartikel-haltige Formulierung konnte nun an den gut ausgebildeten und unbeschädigten Blutgefäßen der Chorion-Allantois-Membranen aufgetragen und getestet werden. Das Testpräparat wurde an insgesamt sechs Eiern getestet, maximal 30 Minuten nach der Öffnung der Eier.

Im Rahmen der HET-CAM Untersuchungen wurden von dem Testpräparat (2,88 mg/ml kolloidale Keratin-Lösung in 0,5 % Hydoxyethylcellulose-Gel) je 200 µl auf die Chorion-Allantois-Membran appliziert und diese für insgesamt 300 Sekunden beobachtet. Während dieser Zeit wurden die Veränderungen an der Membran, hinsichtlich des Zeitpunktes des Auftretens (Irritationsscore, IS) und der Festlegung des Schweregrades zum Ende der Beobachtungszeit, dokumentiert. Hierbei wurden folgende Kriterien bewertet: Hämorrhagien (H) - das Auftreten von Blutungen; Gefäßlyse (L) - das Transparentwerden von Gefäßen und Koagulation (C) - das Stagnieren des Blutflusses als Zeichen der intravasalen Gerinnung. Als Negativkontrolle wurde steriles Wasser und als Positivkontrolle eine 1 %-ige Natriumlaurysulfat-Lösung eingesetzt. Die Berechnung des Irritationsscores (IS) erfolgte anhand der ICCVAM-Kriterien.

Als Bewertungskriterien des ISs wurden folgende Grenzwerte definiert:

| | |
|---|---|
| • IS ≤ 1 | = kein Hinweis für irritatives Potential |
| • IS > 1 und < 4 | = leichtes irritatives Potential |
| • IS > 4 und ≤ 9 | = mäßiges irritatives Potential |
| • IS > 9 | = starkes irritatives Potential |

Der Schweregrad von Veränderungen zum Ende des Beobachtungszeitraums wurde nach den folgenden Kriterien eingestuft:

| | |
|---|---|
| • 0 | = keine Reaktion |
| • 1 | = leichte Reaktion |
| • 2 | = mäßige Reaktion |
| • 3 | = starke Reaktion |

Die Hämorrhagie (H) wurde entsprechend des Ausmaßes von Erythrozytenextravasaten den Kategorien halbquantitativ wie folgt zugeordnet: Einzelne Kapillarblutungen bei morphologisch intakten Kapillaren wurden als leichte, multiple Kapillarblutungen bei morphologisch intakten Kapillaren als mäßige und morphologisch geschädigte Kapillaren mit Kapillarblutungen oder Massenblutungen als schwere Hämorrhagie eingestuft. Vereinzelte transparente Kapillarabschnitte wurden als leichte, die Transparenz von ganzen Kapillaren als mäßige und das Auftreten von kompletten Gefäßlysen als schwere Lyse bewertet. Die Koagulation (C) wurde entsprechend des Ausmaßes von Koagulationsphänomenen den Kategorien halbquantitativ wie folgt zugeordnet: Einzelne Kapillarthrombosen bei morphologisch intakten Kapillaren wurden als leichte, multiple Kapillarthrombosen bei morphologisch intakten Kapillaren als mäßige und geschädigte Kapillaren mit langstreckigen Kapillarthrombosen als schwere C eingestuft. Die eingesetzten Hühnereier wurden nach einer Lagerung für 48 Stunden bei -20°C, hygienisch entsorgt.

**Tab. 6: HET-CAM Untersuchungsdaten für das Testpräparat bestehend aus 2,88 mg/ml kolloidaler Keratinpartikel-Lösung in 0,5 %-igem Hydroxyethylcellulose.**

| | **Sekunden bis Ereignisbeginn** | | | **Irritations-Score (IS)** | | | | **Schweregrad nach 300 Sek.** | | |
|---|---|---|---|---|---|---|---|---|---|---|
| Ei-Nr.: | *Hämorrhagie* | *Gefäßlyse* | *Gerinnung* | *Hämorrhagie* | *Gefäßlyse* | *Gerinnung* | *Gesamt* | *Hämorrhagie* | *Gefäßlyse* | *Gerinnung* |
| 1 | 0 | 0 | 0 | 0,00 | 0,00 | 0,00 | 0,00 | 0 | 0 | 0 |
| 2 | 0 | 0 | 0 | 0,00 | 0,00 | 0,00 | 0,00 | 0 | 0 | 0 |
| 3 | 0 | 0 | 0 | 0,00 | 0,00 | 0,00 | 0,00 | 0 | 0 | 0 |
| 4 | 0 | 0 | 0 | 0,00 | 0,00 | 0,00 | 0,00 | 0 | 0 | 0 |
| 5 | 0 | 0 | 0 | 0,00 | 0,00 | 0,00 | 0,00 | 0 | 0 | 0 |
| 6 | 0 | 0 | 0 | 0,00 | 0,00 | 0,00 | 0,00 | 0 | 0 | 0 |
| Mittelwert | | | | 0,00 | 0,00 | 0,00 | 0,00 | 0,0 | 0,0 | 0,0 |

**Tab. 7: Zusammenfassung der HET-CAM Untersuchungsdaten für das Testpräparat bestehend aus 2,88 mg/ml kolloidaler KeraBods -Lösung in 0,5 % Hydroxyethylcellulose, einer 1%igen Natriumlaurylsulfat-Lösung als Positivkontrolle und Aqua ad injectionem als Negativkontrolle. (C = Koagulation, H = Hämorrhagie, IS = Irritations-Score, L = Gefäßlyse, SG = Schweregrad).**

| **Getestete Präparate** | **IS-H** | **IS-L** | **IS-C** | **IS-Gesamt** | **SG-H** | **SG-L** | **SG-C** | **Irritatives Potential** | |
|---|---|---|---|---|---|---|---|---|---|
| 1 % Natriumlaurylsulfat-Lösung | 2,89 | 0,90 | 6,01 | 9,80 | 1,67 | 0,42 | 3,00 | stark | 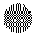 |
| Aqua ad injectionem | 0,00 | 0,00 | 0,00 | 0,00 | 0,00 | 0,00 | 0,00 | kein | 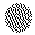 |
| 2,88 mg/ml KeraBods, 0,5% HEC-Gel | 0,00 | 0,00 | 0,00 | 0,00 | 0,00 | 0,00 | 0,00 | kein | 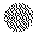 |

Mittels der HET-CAM Untersuchung wurde die toxikologische Wirkung der kolloidal-gelösten Keratinpartikel in einer halbfesten Formulierung getestet. Nach Auftragung des Keratinpartikel-haltigen Testpräparates an der Chorion-Allantois-Membran wurden diese für 300 Sekunden beobachtet und die Ereignisse dokumentiert. Für einen Gehalt von 2,88 mg/ml Keratinpartikel in 0,5%-igem Hydroxyethylcellulose-Gel konnten keine Ereignisse zur Hämorrhagie, Gefäßlyse oder Koagulation beobachtet werden (Tabelle 6+7). Aufgrund dessen scheint der Einsatz von kolloidalen Keratinpartikel in einer Formulierung mit einer Konzentration von bis zu 2,88 mg/ml, zur Anwendung auf der Haut, unbedenklich. Für die verwendete Positivkontrolle, 1%ige Natriumlaurylsulfat-Lösung, konnte hingegen ein starkes irritatives Potential mit einem Irritations-Score von 9,80 ermittelt werden. Des Weiteren konnte für die eingesetzte Negativkontrolle, Aqua ad injectionem, kein irritatives Potential bestimmt werden.

### Penetrationsuntersuchungen mit gelabelten Keratinpartikeln

Für die Isatosäureanhydrid-Markierung der Keratinpartikel wurde der Stoff in einem fünf-molaren Überschuss, ausgehend von einem durchschnittlichen Molekulargewicht der Keratinpartikel von 15 kDa (basierend auf dem Mittelwert der Fraktionen, SDS-PAGE), eingesetzt. Im ersten Schritt wurden 184 mg der Keratinpartikel in 4600 µl des Reaktionspuffers gelöst (40 mg/ml Keratinpartikel, 2,67 mM), bevor 62 µl einer 1 M Isatosäureanhydrid-Lösung zugesetzt wurden (13,35 mM Isatosäureanhydrid im fünf-molaren Überschuss). Nach der Vermengung der Komponenten wurde die Suspension für 25 Minuten bei Raumtemperatur in Dunkelheit inkubiert und im Austausch 1:200 gegen DI-Wasser unter Dunkelheit dialysiert. Nach der Dialyse wurde die Proteinkonzentration des Dialysats mittels Biorad Bradford Assay nach den Angaben des Herstellers bestimmt. Das entstandene Dialysat wurde anschließend als 5%-iger Anteil zu einer DAC-Basiscreme zugesetzt und mit 1% Phenoxyethanol konserviert. Die Keratinpartikel-haltige Formulierung wurde bis zur Verwendung bei 4 °C gelagert und dessen fluoreszierende Eigenschaft vor deren Einsatz mikroskopisch überprüft.

Zur Durchführung der Penetrationsversuche wurde männliche Bauchhaut verwendet. Mittels Korneometrie wurde die Hautintegrität bestimmt. Für die Untersuchungen wurden drei Versuchsbedingungen getestet: A) Haut intakt, 5% kolloidale Keratin-Lösung mit Isatosäureanhydrid-Markierung (Kera-Abz) in Basiscreme DAC und 1% Phenoxyethanol; B) Haut gestrippt, 5% Kera-Abz in Basiscreme DAC und 1% Phenoxyethanol; C) Negativkontrolle, Haut intakt, Basiscreme. Je 20 µl der jeweiligen Formulierung wurden auf ein Hautstück aufgetragen und für 100 Minuten bei 32 °C in der Franzzelle belassen. Anschließend wurden die Hälfte der verbliebenden Formulierung mit einem Wattetupfer abgewischt und aus dem jeweiligen Hautstück, kleinere Stanzen mit einem Durchmesser von 6 mm geschnitten. Die Stanzen wurden im Gefriermikrotom bei -80 °C eingefroren, bevor sagittale Kryoschnitte (bei -20 °C) mit einer Dicke von 10 µm angefertigt und auf einen Objektträger überführt wurden.

Die angefertigten Kryoschnitte wurden im Fluorenzenzmikroskop bei einer 20-fachen Vergrößerung im Farbsetting des DAPI-Filters und im Hellfeld betrachtet. Es wurden Übersichtsbilder zu den einzelnen Versuchsbedingungen aufgenommen, in denen die Formulierung auf der Hautoberfläche zu erkennen war. Die Position der betrachteten Gewebeausschnitte wurde notiert, sodass die Schnitte einer Hämatoxylin-Eosin-Färbung (H&E-Färbung) unterzogen werden konnten. Im Anschluss daran wurde die Gewebeposition der Schnitte erneut aufgesucht und ein weiteres Übersichtsbild im Hellfeld-Modus aufgenommen. Mittels Photoshop CC21 (Adobe, Ireland) wurde ein Overlay aus allen drei Bildern der jeweiligen Gewebeposition erstellt und das Fluoreszenzsignal falsch farbkodiert (grün) (Fig. 2).

Zur Durchführung des Penetrationsversuches musste zunächst die fluoreszierende Eigenschaft der mit Isatosäureanhydrid markierten Keratinpartikel überprüft werden. Hierzu wurde die kolloidale Keratin-Lösung zu 5 % in DAC-Basiscreme aufgenommen und ein Ausstich davon unter dem Fluoreszenzmikroskop betrachtet. Nachdem die fluoreszierende Eigenschaft und die Verteilungsintensität der Keratinpartikel in der Formulierung überprüft wurden, konnte die Penetrationsuntersuchungen durchgeführt werden. Dazu wurde die Formulierung auf intakte und gestrippte Haut aufgetragen und anschließend penetriert. Die angefertigten Kryoschnitte der Haut konnten im Fluoreszenzmikroskop dokumentiert werden, bevor diese einer H&E-Färbung unterzogen wurden. Durch die erneute Dokumentation der Gewebeschnitte konnte ein Overlay erstellt werden, welches die Lokalisierung der aufgetragenen fluoreszierenden Keratinpartikel innerhalb der Hautschichten ermöglichte. Die Keratinpartikel konnten unter allen Versuchsbedingungen ausschließlich in der oberen Schicht des Stratum corneum, dem Stratum disjunction, detektiert werden. Eine Penetration der Keratinpartikel in tiefere Hautschichten konnte im Rahmen der Untersuchungen nicht beobachtet werden. Der Einsatz der Keratinpartikel in kolloidaler Form in eine Formulierung scheint daher unbedenklich.

### Ausschluss des zellulären Uptakes der Keratinpartikel

Um die Risiken des zellulären Uptakes der Keratinpartikel und der damit verbundenen Zell-Keratinpartikel-Interaktion zu untersuchen, wurde eine konfokale Raman-Mikrospektroskopie durchgeführt.

Die Cell Imaging Dishes wurden vor dem Aussäen der HaCaT-Zellen dreimal mit je 1 ml 1 × PBS gewaschen. Zum Aussäen der Zellen wurde eine Zellsuspension, bestehend aus HaCaT-Zellen und 1x Keratinozyten-SFM Medium (mit Zusatz von 10.000 U/ml Penicillin, 10.000 µg/ml Streptomycin, 25 µg/ml Fungizone Antimycotic (Amphotericin B)), mit einer Zellzahl mit 5 × 10⁴ Z/ml hergestellt und pro Cell Imaging Dish wurden jeweils 2 ml ausgesät. Anschließend wurden die Zellen für 72 h bei 37°C und 5% CO₂ kultiviert. Für die Behandlung der HaCaT-Zellen mit der kolloidalen Lösung der KeraBods wurde zunächst ein Medienwechsel durchgeführt. Hierfür wurde das alte Wachstumsmedium aus den Cell Imaging Dishes abgesaugt und durch 2 ml frisches 1x Keratinozyten-SFM Medium ersetzt. Anschließend wurden in je eine Cell Imaging Dish mit enthaltenen HaCaT-Zellen 50 µl Wachstumsmedium als Negativkontrolle, 50 µl Dialysewaschpuffer als Lösungsmittelkontrolle, 200 µg oder 500 µg KeraBods zugegeben. Alle Kulturproben wurden im Anschluss für 48 h bei 37°C und 5% CO₂ inkubiert. Zur Vorbereitung auf die konfokale Raman-Mikrospektroskopie wurde das Medium nach 48 h aus den Cell Imaging Dishes absaugt, bevor die Kulturschalen leicht auf einem Papiertuch ausgeklopft wurden. Im Weiteren wurden die Zellen dreimal mit je 2 ml 1 × PBS gewaschen und beim letzten Waschgang wurde das restliche PBS ebenfalls durch leichtes Klopfen auf ein Papiertuch entfernt. Zur Fixierung der Zellen wurden je 2 ml 4 % Paraformaldehyd pro Kulturschale eingesetzt und für 10 min bei RT inkubieren. Der Paraformaldehyd wurde anschließend absaugt und das restliche Paraformaldehyd durch das Ausklopfen auf ein Papiertuch entfernt. Die Zellen wurden erneut je dreimal mit 2 ml 1 × PBS gewaschen. Für die Untersuchung wurde nach dem letzten Waschschritt nochmals 2 ml frisches 1 × PBS auf die Zellen gegeben und jede Cell Imaging Dish mit Parafilm verschlossen.

Die Durchführung der konfokalen Raman-Mikrospektroskopie wurde durch das Institut für Medizinische Physik und Biophysik der Universität Leipzig (AG Prof. Huster) realisiert. Nach der Übergabe der vorbereiteten Proben erfolgte zeitnah die Aufnahme der Raman-Spektren. Mit Hilfe eines Lasers, der im Wellenlängenbereich von 532 nm arbeitete, wurden die Proben angeregt. Die pixelweise Aufnahme der ableiteten Spektren in allen drei Dimensionen wurde durch das konfokale Raman-Mikroskop detektiert und mittels der WiTec Software Control FOUR und Project FOUR PLUS Software aufgenommen und verarbeitet.

Im Rahmen der konfokalen Raman-Mikrospektroskopie konnten die Spektren der Lipide, der Nuklei und das Zytoplasma innerhalb der HaCaT-Zellen detektiert werden (Fig. 3). Zusätzlich wurde das Raman-Spektrum der kolloidalen Keratin-Lösung separat aufgenommen und zusammenfassend in Fig. 4 dem Spektrum für Lipid, Nukleus und Zytoplasma gegenübergestellt. Mit Hilfe der Daten konnte gezeigt werden, dass es keine wesentlichen Spektralunterschiede zwischen dem Raman-Spektrum der KeraBods und des Zytoplasmas gibt. So verlaufen die ermittelten Spektren in den charakteristischen Bereichen des Phenylalanins (ca. 1000 cm⁻¹), dem C-C Stretch des Amids III (ca. 1250 cm⁻¹) sowie von Amid I (von 1750 cm⁻¹ bis 2000 cm⁻¹) sehr ähnlich. Lediglich im Bereich des CH-Stretchs von ca. 2850 cm⁻¹ bis 3000 cm⁻¹ konnten wesentliche Unterschiede zu den Raman-Spektren der Lipide, des Zytoplasmas und der Nuklei verzeichnet werden (Fig. 4). Des Weiteren zeigen die in Fig. 4 dargestellten optischen Umsetzungen der detektierten Spektren für Lipide, Nuklei und Zytoplasma der mit 500 µg kolloidaler Keratin-Lösung behandelten HaCaT-Zellen, dass auch im zellulären Hintergrund keine Raman-Spektren für die KeraBods erfasst werden konnten. Identische Ergebnisse konnten auch für die mit 200 µg kolloidaler Keratin-Lösung behandelten HaCaT-Zellen erzielt werden (Daten nicht gezeigt). Es kann demnach davon ausgegangen werden, dass die hergestellten KeraBods in kolloidaler Lösung keine Zell-Interaktion mit humanen Keratinozyten bei einer Einsatzkonzentration kleiner als 0,5 mg/ml eingeht.

### Beispiel 3

### Einfluss von Keratinpartikeln auf die Penetrationskinetik eines Modellarzneistoffs

Um den Einfluss von monomerem beta-Keratin auf die Pharmakokinetik von Wirkstoffen nachzuweisen, wurde in einer halbfesten Cremematrix (Monovo^{®} 1 mg/g Creme) gelöstes Mometasonfuroat, als Modellwirkstoff mit 2% Keratinpartikeln versetzt und zur Ausbildung von nicht-kovalenten Proteine-Wirkstoff-Konjugaten homogen verrührt und inkubiert. Zur Objektivierung der pharmakokinetischen Unterschiede wurde eine Diffusionskinetik der Formulierung ohne und mit Beimischung von Keratinpartikeln vergleichend bestimmt.

In der vorliegenden Studie wurde eine HPLC-Methode zur Quantifizierung von Mometasonfuroat in Proben aus der Penetrationsstudie verwendet. Es wurde eine analytische HPLC-UV-Methode entwickelt und validiert. Zur Bestimmung der Wiederfindung wurden dazu 3 Proben der Prüfformulierung mit 1,8 mL Lösungsmittel 1 h lang inkubiert (Laborschüttler, 150 U/min), anschließend zentrifugiert (13.000 U/min, 10 min) und gemessen. Die Wiederfindungsraten wurden mit den experimentell ermittelten Konzentrationen der Prüfgegenstände berechnet. Werte zwischen 95 % und 105 % wurden als unbeeinflusst und damit als korrekt angenommen.

Die Validierung der HPLC-Methode sollte Aufschluss über die Richtigkeit, Präzision und Robustheit der Methode geben. Die Durchführung der Validierung und die Bewertung der Analysemethode wurden gemäß den FDA-Richtlinien durchgeführt. Die Validierung basierte auf einer quantitativen Analyse unter Verwendung externer Standards (Kalibrierungsreihen) und Qualitätskontrollproben. Die Verwendung von Qualitätskontrollproben erlaubte Aussagen über Änderungen in der Probenvorbereitung und -analyse.

Zur Bestimmung der recovery wurden Hauthomogenate von 6 verschiedenen Spendern verwendet. Drei Aliquote jeder Testlösung wurden mit 20 mg Hauthomogenat (n=3) inkubiert. Drei Aliquote jeder Testlösung wurden ohne Haut inkubiert (Referenzlösung, n=3) und ein Aliquot wurde bis zur Messung im Kühlschrank aufbewahrt (Referenz, frisch). Die Inkubation erfolgte auf dem Laborschüttler für 1 Stunde bei 150 U/min. Anschließend wurden die Proben 10 min bei 13.000 U/min zentrifugiert, der Überstand abgenommen, in Braunglasfläschchen mit Glaseinsatz gefüllt und gemessen.

Die Selektivität der Methode wurde für Haut, Tupfermaterial und Formulierungshilfsstoffe des Prüfgegenstandes (Monovo^{®} 1 mg/g Creme) bestimmt. Für die Bestimmung der Selektivität in der Matrix "Haut" wurde Hauthomogenat (ca. 20 mg) von 6 verschiedenen Spendern mit 2 mL MeOH 1 h lang bebrütet (Laborschüttler, 150 U/min), anschließend zentrifugiert (13.000 U/min, 10 min) und gemessen. Zur Bestimmung der Selektivität der Tupferextrakte wurde ein Tupfer 1 h lang mit 2 mL MeOH inkubiert (Laborschüttler, 150 U/min), dann zentrifugiert (13.000 U/min, 10 min) und gemessen.

Zur Bestimmung der Nachweis- und Quantifizierungsgrenze wurde eine Kalibriergerade mit Konzentrationen aufgenommen, die gerade noch und nicht mehr nachweisbar waren (unterer Kalibrierbereich). Es wurde eine lineare Regression der 3 kleinsten bestimmbaren Konzentrationen durchgeführt, wobei das Verhältnis von Konzentration und Signal-Rausch-Verhältnis berechnet wurde. Die Nachweisgrenze (LOD) war die Konzentration mit einem Signal-Rausch-Verhältnis von 3. Die untere Bestimmungsgrenze (LLOQ) war die Konzentration mit einem Signal-Rausch-Verhältnis von 10. Die verwendete HPLC-Methode war innerhalb des Kalibrierbereichs (0,05 - 20 µg/ml für Mometasonfuroat) linear. Die Linearität des Kalibrierbereichs wurde mit gewichteter linearer Regression (Gewicht = 1/C2) geschätzt. Zudem wurde geprüft, ob Mometasonfuroat von einer Probe auf eine andere übertragen werden kann, wenn eine sehr hohe Arzneimittelkonzentration injiziert wird. Daher wurde Methanol dreimal nach dem höchsten Kalibrierungswert (HLOQ) injiziert.

Die Serien von Kalibrierungs- und Qualitätskontrollproben (Cals und QCs) wurden an drei verschiedenen Tagen gemessen. Diese wurden verwendet, um die Richtigkeit, Präzision und Robustheit der Quantifizierungsmethode zu bestimmen. Die Validierungsreihe von Mometasonfuroat umfasste 7 Kalibrierungskonzentrationen (C = 0,05, 0,10, 0,99, 4,97, 9,93, 14,90 und 19,86 µg/ml) und 4 QKs von sehr geringer (LLOQ, C = 0,05 µg/ml), geringer (C = 0,50 µg/ml), mittlerer (C = 7,44 µg/ml) und hoher Konzentration (C = 17,38 µg/ml) an 2 Tagen. An einem Tag wurden drei von jeder Kalibrierungsprobe und 3x drei von jeder QC-Probe gemessen, um die Variabilität innerhalb eines Tages zu bestimmen. Alle Validierungstage wurden zur Bestimmung der Variabilität zwischen den Tagen herangezogen. Die Auswertung erfolgte mittels gewichteter linearer Regression (Gewicht = 1/C2). Die Bewertung der Methode erfolgte anhand der Parameter Mittelwert, Standardabweichung (SD), Variationskoeffizient (CV) und relativer Fehler (RE). Die Abweichung sollte 15% (20% für LLOQ) nicht überschreiten. Die Reproduzierbarkeit wurde durch Messung einer 6-fach unabhängig hergestellten mittleren Cal-Konzentration an einem Tag bestimmt. Die Validierungsparameter Unpräzision und Ungenauigkeit erfüllten die Spezifikationen für die LLOQ von ≤ 15% bzw. 20%. Die Reproduzierbarkeit wurde mit Cal 4, Cnom = 4,97 µg/ml, bewertet. Sie wurde mit Cest = 5,20 ± 0,141 µg/ml bestimmt (CV = 2,7%, entspricht einer Präzision von 97,3%). Zusammenfassend lässt sich sagen, dass die Quantifizierungsmethode für Mometasonfuroat geeignet ist.

Für jede Testformulierung wurden drei Humanhautproben, d. h. von drei verschiedenen Spendern untersucht. Jede Hautprobe ergab drei Stanzbiopsien zu drei verschiedenen Zeitpunkten, d. h. insgesamt 27 Proben je Testformulierung. Für beide Formulierungen wurde die Haut der jeweils gleichen Spender verwendet.

Die Untersuchungen wurden an exzidierter, humaner Brusthaut (3 verschiedene Spender) durchgeführt. Die Gewebeschnitte wurden postoperativ mit Tupfern und isotonischer NaCl-Lösung gereinigt. Das subkutane Fettgewebe wurde mechanisch seziert und verworfen. Zum Untersuchungszeitpunkt wurden die Hautstücke (Ø 20 mm; Fläche: 3,1416 cm²) bei Raumtemperatur vollständig aufgetaut und die Oberfläche mit Tupfern getrocknet.

Die Bestimmung der Hautintegrität erfolgte mittels Corneometer CM 820PC. Werte über 28 Corneometer-Einheiten wurden als geeignet bewertet, andernfalls wurde die Haut verworfen.

Die Untersuchungen wurden in einer Franz'schen-Diffusionszelle aus Glas durchgeführt. Als Akzeptor wurde destilliertes Wasser verwendet. Die einzelnen Hautproben wurden auf Filtergaze aufgetragen und auf die auf 32 °C vorgewärmte Diffusionszelle gelegt. Ein direkter Kontakt zwischen Filtergaze und Akzeptorflüssigkeit musste gewährleistet sein. Die Dicke der Diffusionsschicht wurde durch kontinuierliches Rühren verringert und somit physiologische Verhältnisse simuliert, indem permeierte Wirkstoffanteile stetig entfernt wurden (Sink-Bedingungen). Das System wurde während des Versuchs durch eine Glasabdeckung vor Wasserverdunstung geschützt. Der Versuch begann mit der homogenen Applikation von 20 mg der Prüfformulierung auf die einzelnen Hautproben. Die Inkubationszeit betrug 30, 100 bzw. 300 Minuten. Am Ende der Inkubationszeit wurde die restliche Formulierung mit einem Tupfer entfernt. Danach wurden die Hautproben aus der Diffusionszelle entnommen und drei Biopsien mit einer Größe von Ø 6 mm (0,848 cm²) aus der Haut ausgestanzt. Die Stanzbiopsien wurden anschließend bei -40 °C eingefroren und unter Verwendung eines Kühlmikrotoms horizontal geschnitten. Zur Bestimmung des Konzentrations-Zeit-Profils wurden die einzelnen Hautschichten nach folgendem Schema präpariert: Stratum corneum (SC: 1 Schnitte von je 10 µm); Vitale Epidermis (EP: 2 Schnitt von 20 µm); Dermis (DR: 25 Schnitte von je 40 µm); Rest (verbleibender Stumpf: (St); nicht-penetrierte Anteile: (Tupfer (Tu)); permeierte Anteile (Filtergaze (Ga); Akzeptorflüssigkeit (Ak) (20 ml)).

Die Wirkstoffkonzentrationen in den verschiedenen Hautschnitten wurden unter Berücksichtigung von Schnittdicke, Fläche und applizierter Menge berechnet. Der Anteil der applizierten Dosis wurde anhand der gemessenen Arzneistoffmenge in den Hautkompartimenten, Gaze und Akzeptorflüssigkeit berechnet und ins Verhältnis mit der applizierten Gesamtdosis gesetzt. Der penetrierte Anteil ist die Summe aller Fraktionen der applizierten Dosis in Haut, Gaze und Akzeptor. Die verbleibende Fraktion, die nicht eingedrungene Fraktion, ist der Tupfer. Der permeierte Anteil ist die Summe des Arzneistoffanteils, welcher die Haut vollständig durchdrungen hat, also die in Gaze- und Akzeptorproben nachweisbaren Arzneistoffmengen.

Die Wiederfindung des Arzneistoffs wurde für jeden Hautspender unter Verwendung der Arzneimittelmengen in den Hautabschnitten sowie in den zusätzlichen Proben Tupfer, Gaze und Akzeptorflüssigkeit berechnet. Da 3 Hautspender pro Inkubationszeit verwendet wurden, ergibt sich n = 3 für die Bestimmung der Wiederfindungsrate.

Die Ergebnisse zeigen (Fig. 5), dass die Verwendung von nicht-kovalenten Protein-Wirkstoff-Konjugaten (im vorliegenden Beispiel nicht-kovalente Keratin-Mometasonfuroat-Konjugate) die kutane Bioverfügbarkeit relevant verändert. Durch das höhere Molekulargewicht und den amphiphilen Charakter der Konjugate im Vergleich zum gelösten nicht gebundenen Wirkstoff kommt es zu einer beschleunigten Freisetzung ins Stratum corneum und dadurch zu einem höheren Konzentrationsgradienten, der nach 30 min und 100 min eine relevant höhere Bioverfügbarkeit des Glukokortikoids im Zielkompartiment Korium bedingt. Dies führt zu einer erhöhten Wirkstoffkonzentration nach 30 min von 87,9 µmol/l (43,2%) und nach 100 min von 208,1 µmol/l (66,4%) im Vergleich zum gelösten Wirkstoff (ohne nichtkovalente Keratin-Konjugate). Nach 300 min gleicht sich der Unterschied der Wirkstoff weitgehend aus. Gemessene Unterschiede befinden sich nun im Bereich der Konfidenzintervalle und sind somit nicht relevant. Dieses Beispiel zeigt eindrücklich, dass der Einsatz von nicht-kovalenten Protein-Wirkstoff-Konjugaten einen relevanten Einfluss auf die Pharmakokinetik von Wirkstoffen nehmen kann.

Um nachzuweisen, dass nicht die Anwesenheit von Keratinpartikeln alleinig, sondern das Vorhandensein nicht-kovalenter Keratin-Wirkstoff-Konjugate diese Unterschiede bedingen, wurde mit der identischen halbfesten Cremematrix (Monovo^{®} 1 mg/g Creme) enthaltend den Modellarzneistoff Mometasonfuroat weiterführende Diffusionsversuche in derselben Versuchsanordnung wie oben beschrieben durchgeführt. Dazu wurde die Diffusionskinetik von Mometasonfuroat zu denselben Zeitpunkten jeweils mit der nicht Keratin-beladenen Formulierung ermittelt. Vergleichend dazu wurde in getrennten Versuchen die Koapplikation von Keratin-beladenen Testformulierung ohne Modellwirkstoff 30 min vor sowie 30 min nach der Applikation der Mometasonfuroat-haltigen Testformulierung untersucht. Aus Fig. 6 sind die dabei erhobenen Daten der kutanen Bioverfügbarkeit der einzelnen Testszenarien zu entnehmen. Dabei wird deutlich, dass sich die Diffusionsprofile nicht relevant unterscheiden. Dabei ergeben sich Unterschiede der Wirkstoffkonzentrationen nach 30 min von 10,1 µmol/l (28,4%) bei Vorbehandlung und von 28,4 µmol/l (24,6%) bei Nachbehandlung mit Keratinpartikeln. Auch nach 100 min (69,6 µmol/l (74,4%) vs. 45,2µmol/l (48,2%) und nach 300 min (40,8µmol/l (38,4%) vs. 26,5 µmol/l (24,9%) zeigen sich vergleichbare Daten. Wegen des insgesamt niedrigen Niveaus der absoluten Konzentrationen sind die Unterschiede nicht relevant und der biologisch-anatomischen Variabilität der Hautproben zuzuschreiben. Diese Untersuchungen zeigen, dass für die Einflussnahme zwingend das Vorliegen von nicht-kovalenten Keratin-Wirkstoff-Konjugaten notwendig ist und die alleinige Anwesenheit von Keratinpartikeln keinen relevanten Einfluss auf die Pharmakokinetik des Modellarzneistoffs besitzt.

## Patentansprüche

1. Topische Formulierung enthaltend mindestens ein nicht-kovalentes Keratin-Wirkstoff-Konjugat enthaltend Keratinpartikel des Proteins beta-Keratin und/oder deren Agglomerate aus Federn tierischen Ursprungs und mindestens einen Wirkstoff zur dermatologischen Anwendung.,.

2. Topische Formulierung nach Anspruch 1,
**dadurch gekennzeichnet, dass** das beta-Keratin in seiner Sekundärstruktur als beta-Faltblatt vorliegt.

3. Topische Formulierung nach einem der vorhergehenden Ansprüche, **dadurch gekennzeichnet, dass** die Keratinpartikel und/oder deren Agglomerate in der topischen Formulierung eine Partikelgröße im Bereich von 10 bis 120 µm, bevorzugt von 25 bis 100 µm, gemessen mittels dynamischer Lichtstreuung, aufweisen.

4. Topische Formulierung nach einem der vorhergehenden Ansprüche, **dadurch gekennzeichnet, dass** das Wirkstoff-Träger-System Additiva enthält, bevorzugt ausgewählt aus der Gruppe bestehend aus
• Aminosäuren, Peptide, Proteinen,
• Kohlenhydraten, insbesondere Saccharose, Lactose, Glucose, Fructose, Mannitol, Sorbitol und Süßstoffen wie Saccharin-Natrium, Natriumcyclamat, Aspartam, Stärke und modifizierte Stärke, Cyclodextrine und/oder Mischungen hiervon,
• Ionische Tenside, wie anionische bzw. polyanionische Tenside, insbesondere Natriumdodecylsulfat, Natriumcetylstearylsulfat, Cetylstearylalkohol (emulgierend), Natriumdioctylsulfosuccinat und/oder kationische Tenside, insbesondere Metallseifen und/oder Mischungen hiervon,
• Nichtionische Tenside, insbesondere Fettalkohole und Sterole, Sortitanfettsäureester, Polyoxyethylen-Sorbitanfettsäureester, Polyoxyethylen-Fettsäureglyceride, Macrogol-1000-glycerolmo-nofettsäureester, Polyoxyethylen-Fettsäureester, Polyoxyethylen-Fettalkoholether, Glycerolfettsäureester, Saccharosefettsäureester, Poloxamere und/oder Mischungen hiervon,
• Gelbildner, insbesondere Polyacrylate, Poloxamer, Cellulosederivate, wie Methycellulose, Methylhydroxyproylcellulose Hydroxypropylcellulose, Hydroxyethylcelluloseund/oder Ethylcellulose, Carmellose-Natrium und/oder Mischungen hiervon,
• Verdickungsmittel, insbesondere Tragant, Xantham, arabisches Gummi, Guargalactomannan, Alginate, Bentonit, und/oder Mischungen hiervon,
• Filmbildnern, insbesondere Methacrylsäure-Acrylaten, Polyvidon, Polyvinylalkohol und/oder Mischungen hiervon,
• Polymeren insbesondere Macrogole, Gelatine und/oder Mischungen hiervon,
• Konservierungsstoffe, insbesondere Methyl-4-hydroxybenzoat, Propyl-4-hydroxybenzoat, Sorbinsäure, Kaliumsorbat, Propylenglykol und/oder Mischungen hiervon,
• Antioxidativa, insbesondere Tocopherolacetat, Carotinoide, Flavonoide, Ascorbinsäure und/oder Mischungen hiervon,
• Duftstoffe
• Puffersubstanzen, insbesondere Natriumlactat, Glycolsäure und/oder Mischungen hiervon.

5. Topische Formulierung nach einem der vorhergehenden Ansprüche, **dadurch gekennzeichnet, dass** der mindestens eine Wirkstoff ein lipophiler, hydrophiler oder amphiphiler Wirkstoff ist und bevorzugt ausgewählt ist aus der Gruppe der Immunsuppressiva oder Immunmodulatoren, Antiphlogistika, Antiallergika, Antihistaminika, Antibiotika, Antimykotika, Virustatika, Antiproliferativa, Antineoplastika, Keratolytika, Haarwuchsmitteln, Nageltherapeutika, Antipruriginosa, Lokalanästhetika, Analgetika, Rheologika und Statinen und Mischungen hiervon.

6. Topische Formulierung nach einem der vorhergehenden Ansprüche, **dadurch gekennzeichnet, dass** der oder die enthaltenen Wirk- und Hilfsstoffe pharmazeutisch wirksame Verbindungen zur Behandlung der Psoriasis, Ekzeme, Allergien, Akne, Rosazea und andere entzündliche Hautzustände, benigne, semimaligne oder maligne Hauttumoren, Infektionen durch Pilze, Bakterien oder Viren, Infektion oder Infestation durch Protozoen oder Parasiten, Differenzierungsstörungen der Epidermis, Erkrankungen der Schleimhäute oder Übergangsschleimhaut, Kopfhauterkrankungen, Erkrankungen der Hautanhangsgebilde, Störungen der hämo- oder lymphovaskulären Perfusion, Störungen des Nervensystems, insbesondere Juckreiz- und Schmerzzustände, Bindegewebserkrankungen und Verhornungsstörungen darstellen.

7. Topische Formulierung nach einem der vorhergehenden Ansprüche, **dadurch gekennzeichnet, dass** das in der Formulierung enthaltene mindestens eine nicht-kovalente Keratin-Wirkstoffkonjugat in präformierte Membranen von Kolloiden, insbesondere Liposomen und/oder Cerosomen und/oder Aphrone integrierbar ist.

8. Topische Formulierung nach einem der vorhergehenden Ansprüche, **dadurch gekennzeichnet, dass** das in der Formulierung enthaltene mindestens eine nicht-kovalente Keratin-Wirkstoffkonjugat in die Hüllmembran von extrahierten extrazellulären Vesikeln, insbesondere Exosomen, integrierbar ist.

9. Topische Formulierung nach einem der vorhergehenden Ansprüche, **dadurch gekennzeichnet, dass** das in der Formulierung enthaltene mindestens eine nicht-kovalent gebundene Keratin-Wirkstoff-Konjugat in Nanopartikel aus vollständig oder anteilig amphiphilen Lipiden, insbesondere Phospholipiden oder/und Ceramiden bestehende Nanopartikeln integrierbar ist.

10. Topische Formulierung nach einem der vorhergehenden Ansprüche, **dadurch gekennzeichnet, dass** die Federn tierischen Ursprungs ausgewählt sind aus der Gruppe bestehend aus Federn von Hühnern, Gänsen, Puten, Enten, Truthähnen, Fasanen, Straußen, Nandus, Emus, Wachteln und Mischungen hiervon.

11. Topische Formulierung nach einem der vorhergehenden Ansprüche, **dadurch gekennzeichnet, dass** die dermatologische Anwendung die Steuerung der kutanen und/oder subkutanen Bioverfügbarkeit und/oder zur transdermalen Applikation des mindestens einen Wirkstoffs ist.

12. Topische Formulierung nach einem der vorhergehenden Ansprüche, **dadurch gekennzeichnet, dass** die dermatologische Anwendung an Lebewesen, insbesondere an Menschen und/oder Tieren erfolgt, insbesondere für die Haut, Schleimhaut oder Hautanhangsgebilde, bevorzugt Nägeln und Haaren.

13. Topische Formulierung nach einem der vorhergehenden Ansprüche in Form von
• gasförmige Grundlagen, insbesondere Aerosole und/oder Gasaphronen,
• flüssige Grundlagen, insbesondere Lösungen, Emulsionen, Suspensionen, Schäume und/oder Kolloiden,
• halbfeste Grundlagen, insbesondere Salben, Cremes, Gele, Pasten, Kolloiden und/oder Suppositorien oder
• feste Grundlagen, insbesondere Pulver, Puder, Tabletten, Granulate, Pellets, Kapseln, Pflaster, Wundauflagen und Verbandsmittel, Textilfasern und/oder Inserts.

14. Verwendung von Keratinpartikeln des Proteins beta-Keratin und/oder deren Agglomerate aus Federn tierischen Ursprungs zur Herstellung von Arzneimitteln für die dermatologische Anwendung.

15. Verwendung nach Anspruch 14,
**dadurch gekennzeichnet, dass** durch Kontakt mit mindestens einem Stoff ausgewählt aus der Gruppe der Antigene, Allergene und Kombinationen hiervon, die nach Applikation auf die Haut ein nicht-kovalentes Keratin-Stoff-Konjugat bilden und dadurch die Penetrationskinetik bzw. Bioverfügbarkeit des mindestens einen Wirkstoffs steuerbar ist.
